# EUROPEAN PATENT APPLICATION

(11) **EP 2 123 243 A1**
(43) Date of publication of application: **25.11.2009**
(21) Application number: 08702959.1
(22) Date of filing: 08.01.2008
(51) Int. Cl.: A61F 13/15, A61F 13/472, A61F 13/56

(54) **ABSORPTIVE ARTICLE**

(30) Priority: 22.01.2007 JP 2007011643
(71) Applicant: UNI-CHARM CORPORATION, Shikokuchuo-shi, Ehime 799-0111 (JP)
(72) Inventor: KUDO, Jun, Kanonji-shi Kagawa 769-1602 (JP); SHIMOE, Nariaki, Minato-ku Tokyo 108-8575 (JP); KINOSHITA, Hideyuki, Kanonji-shi Kagawa 769-1602 (JP); HASHINO, Akira, Kanonji-shi Kagawa 769-1602 (JP)
(74) Representative: Sperling, Rüdiger
(86) International application number: PCT/JP2008/050079
(87) International publication number: WO 2008/090756

(57) **Abstract**

An absorbent article 1 that is unlikely to be detached from an undergarment 90 at the time of pulling-up operation of an absorbent body 20 in wearing the absorbent article 1, and has excellent stability of adhesion to the undergarment 90 is provided.

An absorbent article 1 that is worn on a human body and used, including an absorbent body 20 having a fluid-absorbing member 22 that absorbs fluid and having a longitudinal direction, a width direction, and a thickness direction; a main body 10 whose face on the human body side is overlapped with the absorbent body 20 in the thickness direction; and adhesive sections 31, 31 for adhering the main body 10 to an undergarment, and the adhesive sections 31, 31 being formed on a face on the opposite side of the face on the human body side of the main body 10, wherein a part 25 of the absorbent body 20 in the longitudinal direction is joined to a predetermined portion 10g of the main body 10, and an end section 20b of the absorbent body 20 in the longitudinal direction can be moved separably from the main body 10 as a free end with respect to the part 25, and wherein the adhesive sections 31, 31 are included continuously spanning, in the longitudinal direction, an end 10h of the predetermined portion 10g, the end 10h being on the free end side in the longitudinal direction.

## Description

### Technical Field

The present invention relates to absorbent articles.

### Background Art

As an example of an absorbent article that absorbs fluid discharged from a human body such as menstrual blood, a sanitary napkin is known. Recently, the types of sanitary napkins have increased, and as one type of the sanitary napkin, there is a sanitary napkin including (1) a main body that is fixed to an undergarment and (2) an absorbent body that includes a fluid-absorbing member, such as a pulverized pulp that absorbs fluid, and is overlapped with the surface of the main body, in which a front end section of the absorbent body in a longitudinal direction is joined to a predetermined portion of the main body, and a rear end section can be moved separably from the main body as a free end with the front end section serving as a fulcrum (see JP-A-2002-159534, for example).

### Disclosure of Invention

### Problems to be Solved by the Invention

At the time of use, the main body is attached and fixed to an inner face (i.e., a face on human body side) of an undergarment by a "displacement-preventing adhesive section" provided on a back face of the main body while the absorbent body is arranged so that the longitudinal direction thereof is aligned with a front-and-rear direction of a human body. Then, in a state where the undergarment is worn, a rear end section of the absorbent body is pulled up so that the absorbent body is placed in a groove of buttocks and the like. Accordingly, the absorbent body is sandwiched and fixed in the groove of the buttocks and the like.

In the absorbent article of this Patent Document 1, the displacement-preventing adhesive section is formed separately in two sections, that are, a front end section and a rear end section of the main body. The displacement-preventing adhesive section in the front end section of these two end sections is formed only in a region forward of the rear end of the predetermined portion (a portion to which the front end section of the absorbent body is joined) of the main body. That is to say, this displacement-preventing adhesive section is not included continuously spanning the rear end of the predetermined portion from front to rear.

Thus, due to a pulling force that acts on the rear end of the predetermined portion at the time of pulling up the rear end section of the absorbent body as described above, this displacement-preventing adhesive section is detached easily and with relatively little resistance, starting from the rear end. Accordingly, the absorbent article has a problem in terms of the stability of adhesion to the undergarment.

The present invention was made in view of the foregoing conventional problem, and it is an advantage thereof to provide an absorbent article that is unlikely to be detached from the undergarment at the time of pulling-up operation of the absorbent body in wearing the absorbent article, and has excellent stability of adhesion to the undergarment.

### Means for Solving the Problems

A main aspect of the invention for achieving the foregoing object is
an absorbent article that is worn on a human body and used, including:
an absorbent body having a fluid-absorbing member that absorbs fluid and having a longitudinal direction, a width direction, and a thickness direction;
a main body whose face on the human body side is overlapped with the absorbent body in the thickness direction; and
an adhesive section for adhering the main body to an undergarment, the adhesive section being formed on a face on the opposite side of the face on the human body side of the main body,
wherein a part of the absorbent body in the longitudinal direction is joined to a predetermined portion of the main body, and an end section of the absorbent body in the longitudinal direction can be moved separably from the main body as a free end with respect to the part, and
wherein the adhesive section is included continuously spanning, in the longitudinal direction, an end of the predetermined portion, the end being on the free end side in the longitudinal direction.

Other features of the invention will become clear by the description of the present specification and the accompanying drawings.

### Effects of the Invention

According to the present invention, it is possible to provide an absorbent article that is unlikely to be detached from an undergarment at the time of pulling-up operation of an absorbent body in wearing the absorbent article, and has excellent stability of adhesion to the undergarment.

### Brief Description of Drawings

Fig. 1 is a plan view of a surface side of an absorbent article 1 in an unfolded state.
Fig. 2 is a plan view of a back face side of the absorbent article 1 in an unfolded state.
Fig. 3A is a cross-sectional view taken along line A-A in Fig. 1, and Fig. 3B is a cross-sectional view taken along line B-B in Fig. 1.
Fig. 4 is a cross-sectional view taken along line IV-IV in Fig. 1.
Fig. 5 is a perspective view of the absorbent article 1.
Fig. 6 is a perspective view of the absorbent article 1, for illustrating a state of the absorbent article 1 in use.
Fig. 7 is a perspective view of the absorbent article 1, for illustrating a state of the absorbent article 1 in use.
Fig. 8 is a perspective view of the absorbent article 1, for illustrating a state of the absorbent article 1 in use.
Fig. 9 is a plan view of a top absorbent body 20 in an unfolded state.
Fig. 10A is a cross-sectional view taken along line A-A in Fig. 9, Fig. 10B is a cross-sectional view taken along line B-B in Fig. 9, Fig. 10C is a cross-sectional view taken along line C-C in Fig. 9, and Fig. 10D is a cross-sectional view taken along line D-D in Fig. 9.
Fig. 11 is a diagram illustrating the manner in which the absorbent article 1 is wrapped.
Figs. 12A and 12B are schematic diagrams for illustrating problems at the time of wearing the absorbent article 1, and are vertical cross-sectional views taken along the center of a human body, showing a state in which an absorbent article 1 of a comparative example is worn on the human body.
Fig. 13 is a vertical cross-sectional view taken along the center of the human body, showing a state in which an absorbent article 1 of present embodiment is worn on the human body.
Fig. 14A is a plan view of an absorbent article 1 of first embodiment, and Fig. 14B is a cross-sectional view taken along line B-B in Fig. 14A.
Figs. 15A to 15C are explanatory diagrams of application patterns of a hot-melt adhesive HMA applied to a permanent joining portion 10g.
Fig. 16 is an explanatory diagram of an effect of the absorbent article 1 of first embodiment.
Fig. 17A is a plan view of an absorbent article 1 of a modified example of the first embodiment, and Fig. 17B is a cross-sectional view taken along line B-B in Fig. 17A.
Figs. 18A and 18B are explanatory diagrams of a reason why the first embodiment is superior to the modification thereof.
Fig. 19A is a plan view of an absorbent article 1 of second embodiment, and Fig. 19B is a cross-sectional view taken along line B-B in Fig. 19A.
Fig. 20 is an explanatory diagram of an effect of the absorbent article 1 of the second embodiment.
Fig. 21A is a plan view of an absorbent article 1 of a modified example of the second embodiment, and Fig. 21B is a cross-sectional view taken along line B-B in Fig. 21A.
Fig. 22A is a plan view of an absorbent article 1 of third embodiment, and Fig. 22B is a cross-sectional view taken along line B-B in Fig. 22A.
Fig. 23A is an explanatory diagram of a permanent joining portion 10g of another embodiment.
Fig. 23B is an explanatory diagram of a permanent joining portion 10g of another embodiment.
Fig. 24 is an explanatory diagram of an absorbent article 1 of another embodiment, showing a plan view of the absorbent article 1 in an unfolded state.
Fig. 25 is an explanatory diagram of an absorbent article 1 of another embodiment, showing a plan view of the absorbent article 1 in an unfolded state.
Fig. 26 is an explanatory diagram of an absorbent article 1 of another embodiment, showing a plan view of the absorbent article 1 in an unfolded state.
Fig. 27 is an explanatory diagram of an absorbent article 1 of another embodiment, showing a perspective view of the absorbent article 1 in an unfolded state.
Fig. 28 is an explanatory diagram of an absorbent article 1 of another embodiment, showing a perspective view of the absorbent article 1 in an unfolded state.

### List of Reference Numerals

1 absorbent article, 1a front end section, 1b rear end section, 10 base absorbent body (main body), 10a front end section, 10b rear end section, 10e rear end edge, 10f rear end edge (end edge), 10fb end (end positioned closest to free end side), 10fl left end (end in width direction of the end edge), 10fr right end (end in the width direction of the end edge), 10i front end edge, 10h rear end (end on the free end side), 10g permanent joining portion (predetermined portion), 12 absorbent body base material, 12a pulverized pulp, 14 surface sheet, 20 top absorbent body (absorbent body), 20a front end section, 20b rear end section (end section), 20c center section, 22 pulverized pulp (fluid-absorbing member), 22f thin-walled section, 23 intermediate sheet, 24 shape retaining sheet, 24c portion, 24d portion, 24e end section, 25 front-side sealed section (a part), 25a pick-up section, 26 rear-side sealed section, 26a pick-up section, 26b portion, 27 hook member, 30 back face sheet, 30a front end section, 30b rear end section, 30c back face, 31 displacement-preventing adhesive section (adhesive section), 31a frond end edge, 31b rear end edge, 31' displacement-preventing adhesive section, 32 wing section, 33 second displacement-preventing adhesive section, 34 protection sheet, 35 protection sheet, 36 wrapping sheet, 36c edge section, 38 lead tape, 90 undergarment, R overlapping section, W width, W1 distance, W2 width, CL center line, Z position assumed to abut against a bodily discharge opening

### Best Mode for Carrying Out the Invention

At least the following matters will be made clear by the explanation in the present specification and the description of the accompanying drawings.

An absorbent article that is worn on a human body and used, including:
an absorbent body having a fluid-absorbing member that absorbs fluid and having a longitudinal direction, a width direction, and a thickness direction;
a main body whose face on the human body side is overlapped with the absorbent body in the thickness direction; and
an adhesive section for adhering the main body to an undergarment, the adhesive section being formed on a face on the opposite side of the face on the human body side of the main body,
wherein a part of the absorbent body in the longitudinal direction is joined to a predetermined portion of the main body, and an end section of the absorbent body in the longitudinal direction can be moved separably from the main body as a free end with respect to the part, and
wherein the adhesive section is included continuously spanning, in the longitudinal direction, an end of the predetermined portion, the end being on the free end side in the longitudinal direction.

According to this absorbent article, the adhesive sections for adhering the main body to the undergarment are included continuously spanning, in the longitudinal direction, the end of the predetermined portion on the free end side. Thus, even if a pulling force acts on the end of the predetermined portion on the free end side, the adhesive sections effectively counteract the pulling force and prevent the main body from being detached from the undergarment. Accordingly, the absorbent article becomes unlikely to be detached from the undergarment at the time of pulling-up operation of the absorbent body in wearing the absorbent article, and has excellent stability of adhesion to the undergarment.

In the absorbent article, it is preferable that the adhesive sections are included continuously spanning, in the longitudinal direction, at least an end that is positioned closest to the free end side in the end.

According to this absorbent article, the absorbent article can be effectively prevented from being detached from the undergarment at the time of pulling-up operation of the absorbent body in wearing the absorbent article. The reason for this is as follows. The pulling force tends to intensively act on the end that is positioned closest to the free end side in the end. The detachment of the absorbent article from the undergarment starts at an area corresponding to the end that is positioned closest to the free end, and proceeds therefrom. In this regard, since the adhesive section is included continuously spanning, in the longitudinal direction, the end that is positioned closest to the free end side, it is possible to suppress the occurrence of the detachment of the absorbent article from the undergarment starting at that area. Accordingly, the absorbent article can be effectively prevented from being detached from the undergarment at the time of the pulling-up operation of the absorbent body in wearing the absorbent article.

In the absorbent article, it is preferable that the end of the predetermined portion on the free end side defines an end edge having a predetermined width in the width direction orthogonal to the longitudinal direction, and
the adhesive section is included continuously spanning the end edge in the longitudinal direction.

According to this absorbent article, the adhesive section is included continuously spanning, in the longitudinal direction, the end edge of the predetermined portion on the free end side. Thus, even in a case where a pulling force acts on the end edge, the adhesive section effectively counteracts the pulling force and prevents the main body from being detached from the undergarment. Accordingly, the absorbent article becomes unlikely to be detached from the undergarment at the time of the pulling-up operation of the absorbent body in wearing the absorbent article, and has excellent stability of adhesion to the undergarment.

In the absorbent article, it is preferable that the adhesive section is disposed at least outside the end edge in the width direction.

According to this absorbent article, the adhesive section is disposed at least outside the end edge in the width direction. Thus, in the case where a pulling force acts on the predetermined portion, the adhesive section can effectively prevent both end sections in the width direction of the main body from being bent back and separated from the undergarment.

In the absorbent article, it is preferable that an absorbent body and a main body are overlapped while matching their center in the width direction, and
the adhesive section is axisymmetrically disposed relative to a center line of the absorbent body in the width direction.

According to this absorbent article, the adhesive section is axisymmetrically disposed relative to the center line in the width direction of the absorbent body. Thus, the adhesive section can counteract the pulling force in a well-balanced manner in the width direction.

In the absorbent article, it is preferable that the adhesive section is included continuously spanning, in the width direction, an end of the end edge in the width direction.

According to this absorbent article, the adhesive section is included continuously spanning, in the width direction, the end in the width direction of the end edge. Thus, even in a case where a pulling force acts on the end edge, the adhesive section more effectively counteracts the pulling force and prevents the main body from being detached from the undergarment. Accordingly, the absorbent article becomes unlikely to be detached from the undergarment at the time of the pulling-up operation of the absorbent body in wearing the absorbent article, and has excellent stability of adhesion to the undergarment.

In the absorbent article, it is preferable that the adhesive section is included continuously spanning the end edge in the width direction.

According to this absorbent article, the adhesive section is provided also in a center portion in the width direction of the end edge. Thus, a center portion in the width direction of the main body can be effectively prevented from being detached from the undergarment and lifted when a pulling force is applied to the predetermined portion.

In the absorbent article, it is also possible that the absorbent body has one end section and another end section with respect to the longitudinal direction; and a part is the one end section, and an end section serving as a free end is the other end section.

In the absorbent article, it is also possible that at the time of wearing the absorbent article on the human body, in a state in which the main body is adhered to the undergarment by the adhesive section, the absorbent body is pulled in the longitudinal direction, with the end section of the free end serving as a point where force acts.

### === First Embodiment ===

### <Absorbent Article 1>

An absorbent article 1 is, for example, a sanitary napkin. In the following description, the side that is brought into contact with a human body is referred to as a surface side, the side that is brought into contact with an undergarment 90 is referred to as a back face side, an end section that is positioned on the front side of the human body when worn is referred to as a front end section, and an end section that is positioned on the rear side is referred to as a rear end section. Furthermore, the normal direction of the surface or the back face of the absorbent article 1 is also referred to as the thickness direction.

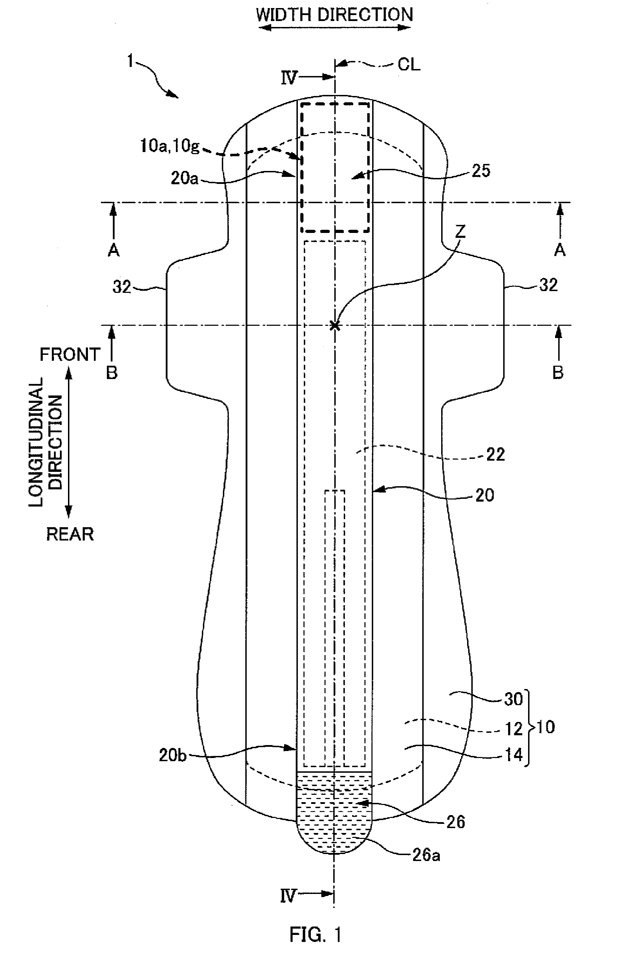

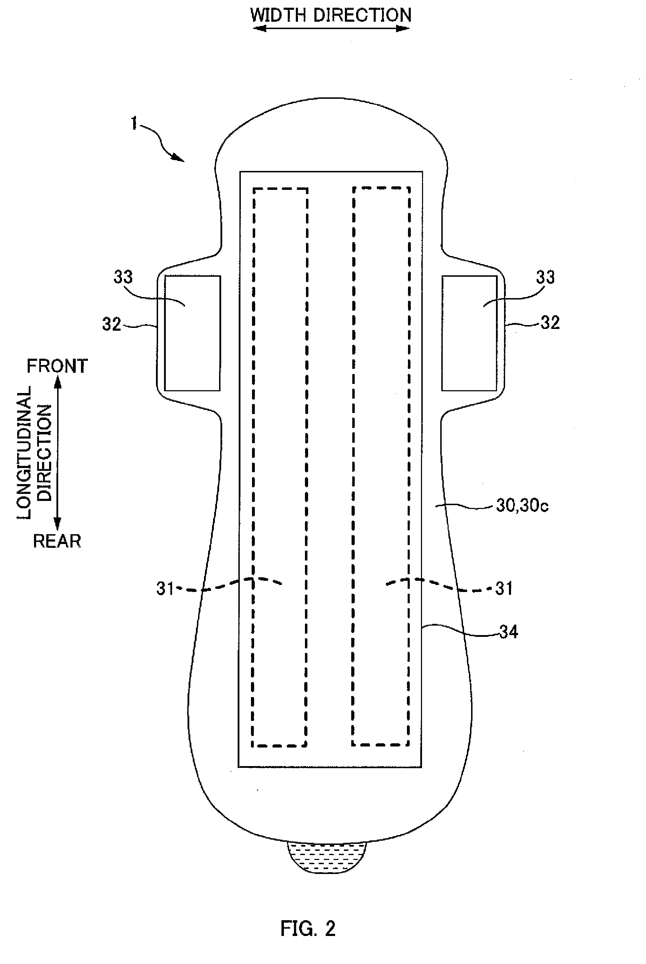

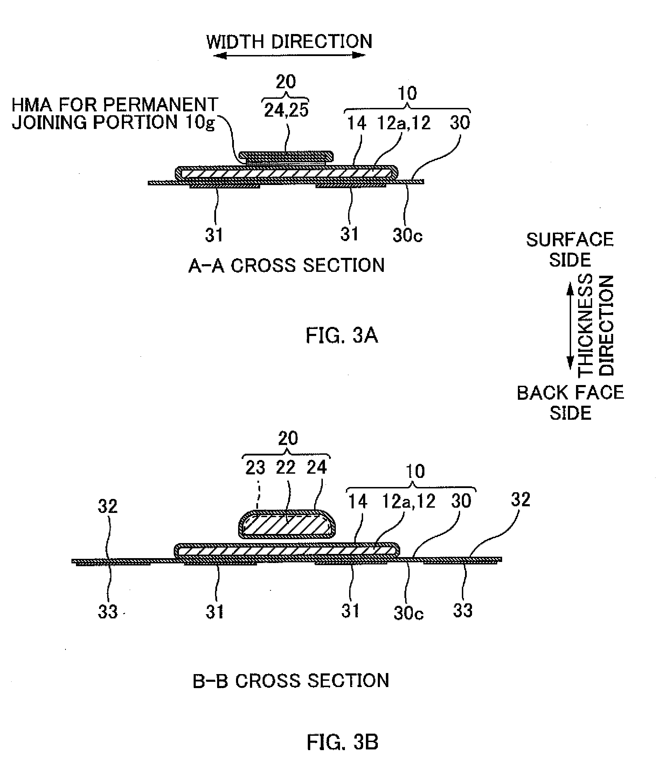

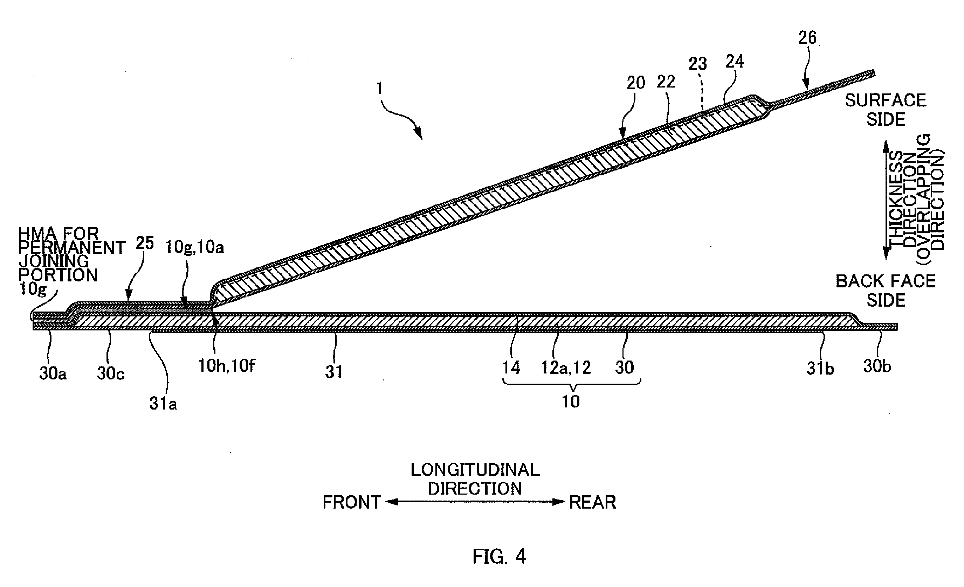

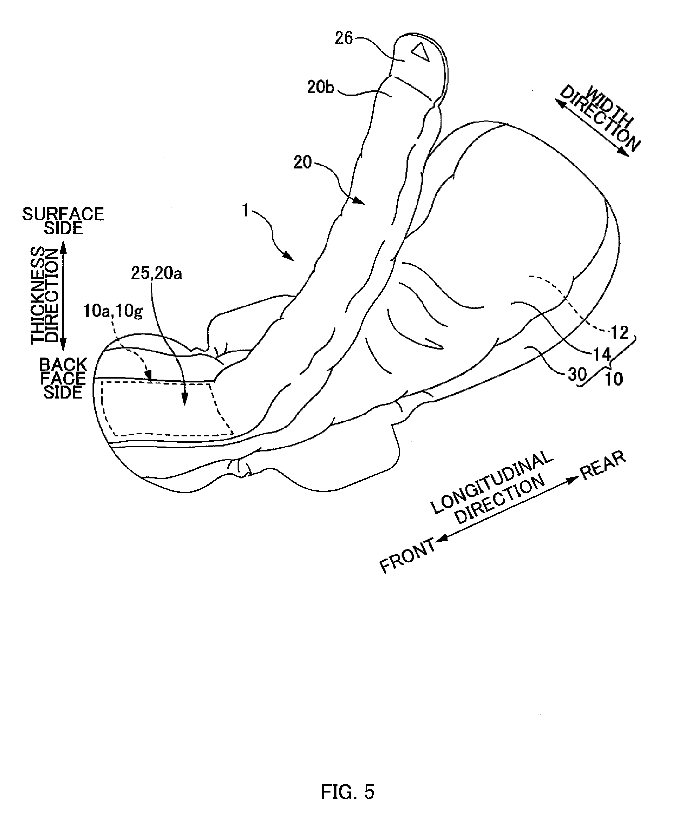

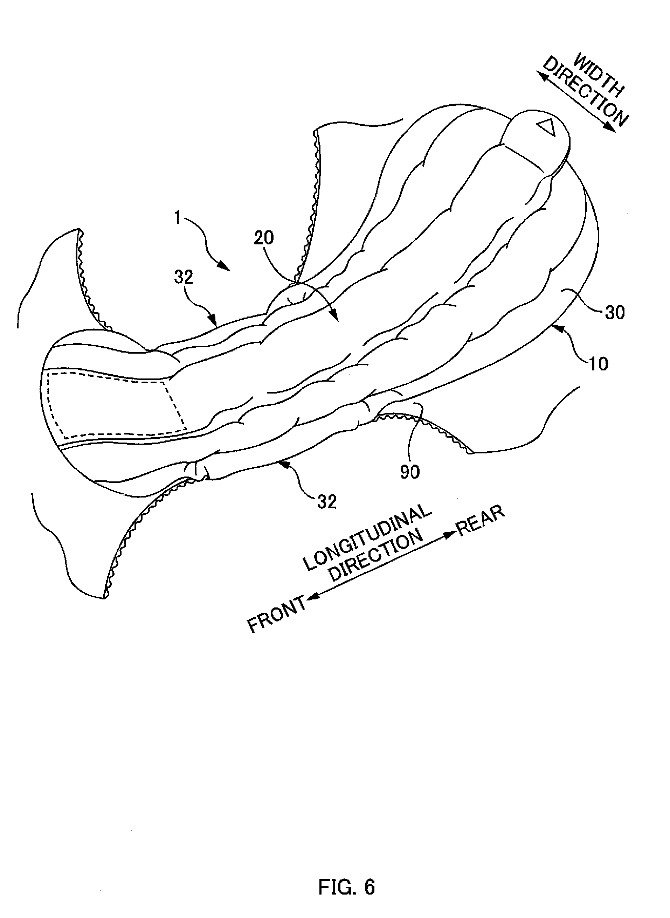

Fig. 1 is a plan view of the surface side of the absorbent article 1 in an unfolded state. Fig. 2 is a plan view of the back face side of the absorbent article 1 in an unfolded state. Figs. 3A and 3B are cross-sectional views taken along lines A-A and B-B in Fig. 1, respectively. Fig. 4 is a cross-sectional view taken along line IV-IV in Fig. 1. Fig. 5 is a perspective view of the absorbent article 1. Figs. 6 to 8 are perspective views of the absorbent article 1 for illustrating a state of the absorbent article 1 in use.

As shown in Figs. 1 and 4, the absorbent article 1 has a shape that is generally elongated in a predetermined direction. As shown in Fig. 5, the absorbent article 1 includes a substantially rectangular base absorbent body 10 (corresponding to a main body) for absorbing fluid such as menstrual blood and a top absorbent body 20 (corresponding to an absorbent body) that is overlapped with the surface of the base absorbent body 10 and disposed in the center in the width direction of the base absorbent body 10 along the longitudinal direction.

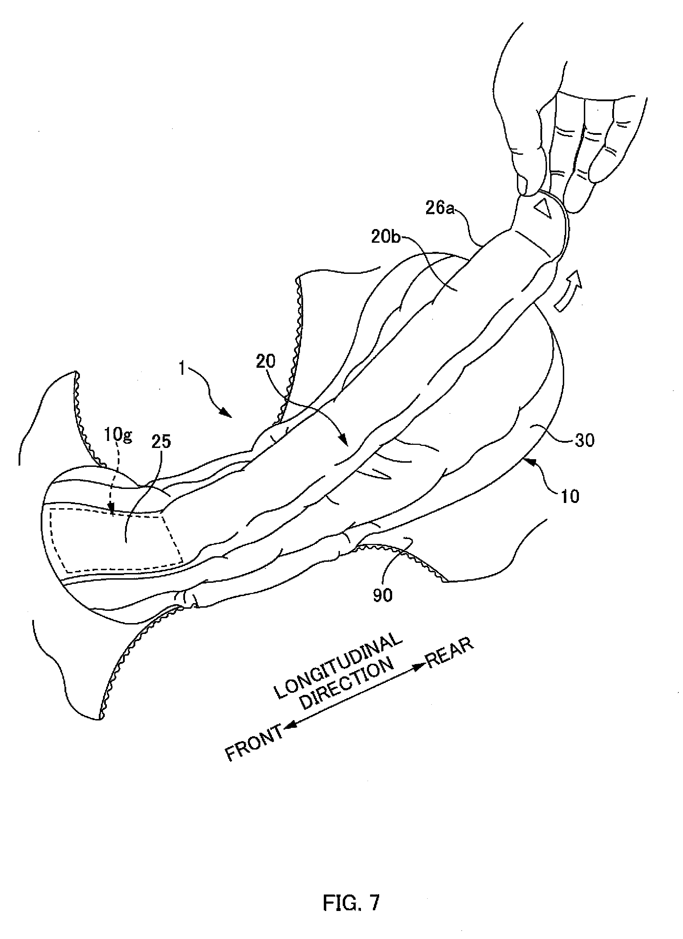

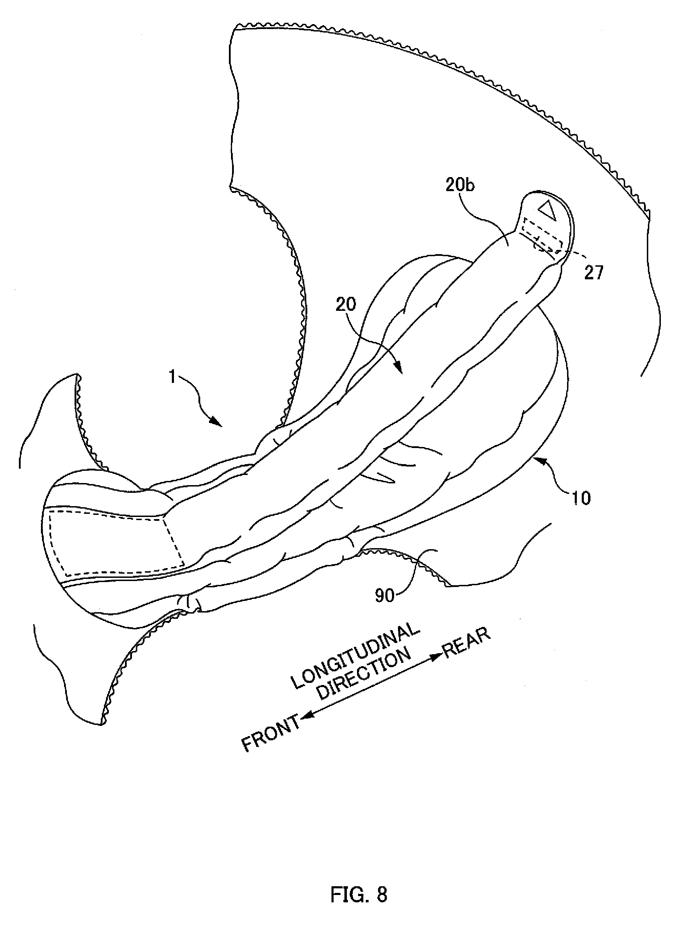

Here, in the top absorbent body 20, a front end section 20a is joined to a front end section 10a of the base absorbent body 10, while a rear end section 20b is a free end that can be separated from the base absorbent body 10 with the front end section 20a as a fulcrum. That is to say, the rear end section 20b of the top absorbent body 20 can move in the thickness direction, a direction in which the top absorbent body 20 is overlapped with the base absorbent body 10, or in the longitudinal direction from front to rear. Thus, at the time of using the absorbent article 1, as shown in Fig. 6, the base absorbent body 10 is attached and fixed to the inner face of the undergarment 90 while arranged so that the longitudinal direction of the top absorbent body 20 is aligned along the front-and-rear direction of the human body. Then, in a state where the undergarment 90 is worn, as shown in Fig. 7, the rear end section 20b of the top absorbent body 20 is pulled up and thereby the top absorbent body 20 is placed in the groove of the buttocks and the like. In this state, as shown in Fig. 8, the rear end section 20b of the top absorbent body 20 is engaged and fixed to the undergarment 90 and the top absorbent body 20 is worn on the human body. And fluid that is discharged from the groove is absorbed mainly by the top absorbent body 20.

It should be noted that, in this worn state, as shown in Fig. 1, a position Z assumed to abut against a bodily discharge opening, at which the bodily discharge opening (i.e., an area from which the fluid is discharged) is supposed to abut against the absorbent article 1, is positioned on a center line CL in the width direction of the absorbent article 1, and on the front side from the center in the longitudinal direction. That is, in the absorbent article 1, the length from the position Z assumed to abut against the bodily discharge opening to the rear side is longer than the length from the position Z assumed to abut against the bodily discharge opening to the front side.

Hereafter is a detailed description of each constituent element of the absorbent article 1.

### <Base Absorbent Body 10>

As shown in Figs. 3A, 3B, and 4, the base absorbent body 10 includes an absorbent body base material 12 in the form of an approximately flat rectangular shape in which a pulverized pulp 12a containing a superabsorbent polymer is wrapped in a fluid-permeable sheet (not shown) such as tissue paper, a surface sheet 14 that is included to cover at least the entire face on the surface side of the absorbent body base material 12, and a back face sheet 30 for preventing leakage of fluid that has been absorbed by the absorbent body base material 12 to the back face side.

The surface sheet 14 is, for example, a fluid-permeable sheet that is substantially rectangular and has a shape approximately similar to the shape of the absorbent body base material 12 viewed from above. The surface sheet 14 is made of a material such as an appropriate nonwoven fabric. For example, a spunlaced nonwoven fabric made of a cellulose fiber such as rayon, a synthetic resin fiber or the like, and an air-through nonwoven fabric made of the synthetic resin fiber.

The back face sheet 30 is, for example, a fluid-impermeable sheet made of a material such as polyethylene or polypropylene, and the shape of the back face sheet 30 is same length as the absorbent body base material 12 in the longitudinal direction, and wider than the absorbent body base material 12 in the width direction. As shown in Fig. 4, in a state where the absorbent body base material 12 is placed on the surface of the back face sheet 30, the back face sheet 30 is joined to the surface sheet 14 at least at a front end section 30a and a rear end section 30b, and thus the absorbent body base material 12 is held between the back face sheet 30 and the surface sheet 14.

It should be noted that, as shown in Figs. 2 to 4, "displacement-preventing adhesive section 31" is included on a back face 30c of the back face sheet 30 for attaching and fixing the absorbent article 1 to the undergarment 90, so that the absorbent article 1 does not slip away from the disposed position after being disposed on the inner face of the undergarment 90. This displacement-preventing adhesive section 31, for example, is a hot-melt adhesive that has been applied in a predetermined area in the back face 30c of the back face sheet 30. And, a preferable formation area of the displacement-preventing adhesive section 31, that is, the aforementioned predetermined area is determined depending on the positional relationship between a permanent joining portion 10g, at which the top absorbent body 20 is permanently joined to the base absorbent body 10. The preferable formation area will be described later.

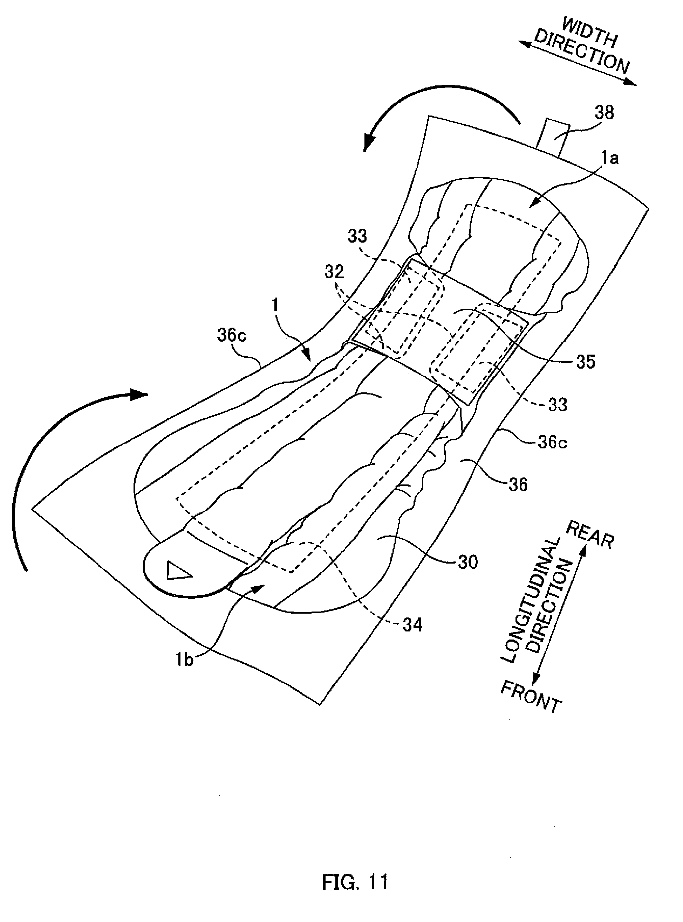

In order to further strengthen the displacement prevention between the undergarment 90 and the absorbent article 1, as shown in Figs. 2 and 3B, wing sections 32 are formed extended toward the outside in the width direction at both ends in the width direction of the back face sheet 30, and "displacement-preventing adhesive sections 33" (hereinafter referred to as "second displacement-preventing adhesive sections") are also included on the back face of these wing sections 32. More specifically, for example, a hot-melt adhesive is applied also to the back faces of the wing sections 32. These wing sections 32 are folded back to the outside as shown in Fig. 6, and attached and fixed to the outer face of the undergarment 90 by the second displacement-preventing adhesive sections 33. It should be noted that before use, protection sheets 34 and 35 for protecting the adhesive properties are attached to the displacement-preventing adhesive sections 31 and 33 (see Figs. 2 and 11) . The protection sheets 34 and 35 are removed immediately before the absorbent article 1 is used.

### <Top Absorbent Body 20>

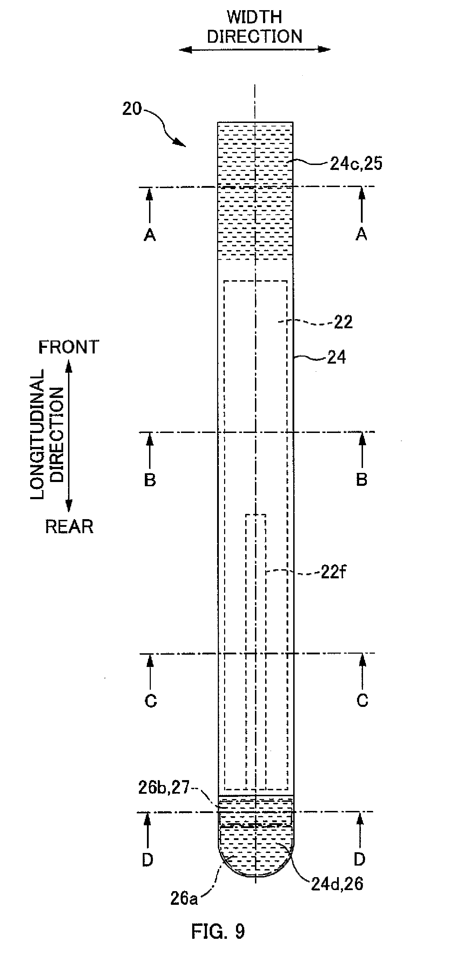

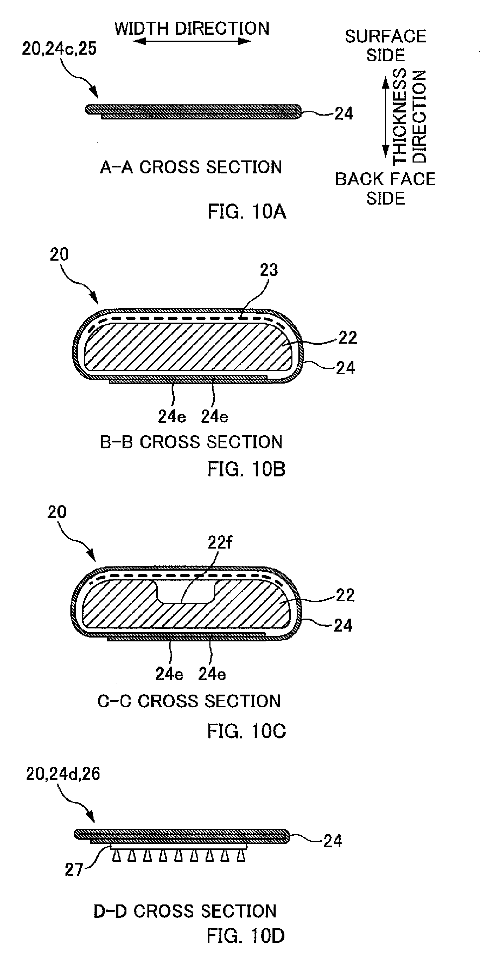

Fig. 9 is a plan view of the top absorbent body 20 in an unfolded state. Further, Figs. 10A to 10D are cross-sectional views taken along lines A-A, B-B, C-C, and D-D in Fig. 9 respectively.

As shown in Figs. 9, 10B, and 10C, the top absorbent body 20 includes a pulverized pulp 22 (corresponding to a fluid-absorbing member that absorbs fluid) containing a superabsorbent polymer, an intermediate sheet 23 that is disposed closer to the surface side than the pulverized pulp 22, and a shape retaining sheet 24 for collectively wrapping around the pulverized pulp 22 and the intermediate sheet 23, thereby retaining these pulverized pulp 22 and the like in the shape of a long article that is long in the longitudinal direction.

The intermediate sheet 23 is a rectangular fluid-permeable sheet with better properties of drawing in fluid than the shape retaining sheet 24. For example, a sheet made of the same material as, but with higher density, the shape retaining sheet 24 is used as the intermediate sheet 23.

The shape retaining sheet 24 is a fluid-permeable sheet. The shape retaining sheet 24 is made of a material such as an appropriate nonwoven fabric. For example, a spunlaced nonwoven fabric made of a cellulose fiber such as rayon, a synthetic resin fiber or the like, and an air-through nonwoven fabric made of the synthetic resin fiber is used. The shape viewed from above of the shape retaining sheet 24 that has been unfolded flat as a sheet is substantially rectangular. End sections 24e on both ends in the width direction of the shape retaining sheet 24 are overlapped and joined to each other via a hot-melt adhesive, so that the shape retaining sheet 24 becomes a tubular shape as shown in Figs. 10B and 10C. In this state, the pulverized pulp 22 and the intermediate sheet 23 are contained inside the tubular body along the longitudinal direction.

Furthermore, a front end portion 24c and a rear end portion 24d in the longitudinal direction of the shape retaining sheet 24 shown in Fig. 9 are each folded in a state without the pulverized pulp 22 or the intermediate sheet 23, as shown in Figs. 10A and 10D, and embossing is performed in a state where an adhesive (not shown) is interposed in the folded portions 24c and 24d. As a result, in a front end section and a rear end section in the longitudinal direction of the shape retaining sheet 24, thin sealed sections 25 and 26 for sealing these end sections are formed in substantially rectangular shapes when viewed from above (see Fig. 9).

Incidentally, the above embossing is performed by a processing target being sandwiched/pressed with a pair of sandwiching/pressing members (not shown) that oppose each other. That is, protrusions are formed in one sandwiching/pressing member of the pair of sandwiching/pressing members, and a face opposing the protrusions in the other sandwiching/pressing member is formed flat. Here, since the tips of the protrusions are formed flat, concave sections with flat bottom section are compressed and formed in the processing target at the time it is sandwiched with the pair of sandwiching/pressing members, and these concave sections become embossed.

As shown in Fig. 1, this sort of top absorbent body 20 has substantially the same length as the base absorbent body 10 in the longitudinal direction, but it is narrower than the base absorbent body 10 in the width direction. And in the center of the base absorbent body 10 in the width direction, the top absorbent body 20 overlaps the base absorbent body 10 while matching their center lines CL in the width direction. As shown in Figs. 1, 4, and 5, the front-side sealed section 25 of the top absorbent body 20 is firmly and permanently joined to the front end section 10a of the base absorbent body 10, however other portions are basically not permanently joined, for example, the rear-side sealed section 26 can be spaced apart from the base absorbent body 10. More specifically, the top absorbent body 20 is bent at the rear end edge of the front-side sealed section 25 and is rotatable in the front-and-rear direction with the sealed section 25 as a fulcrum.

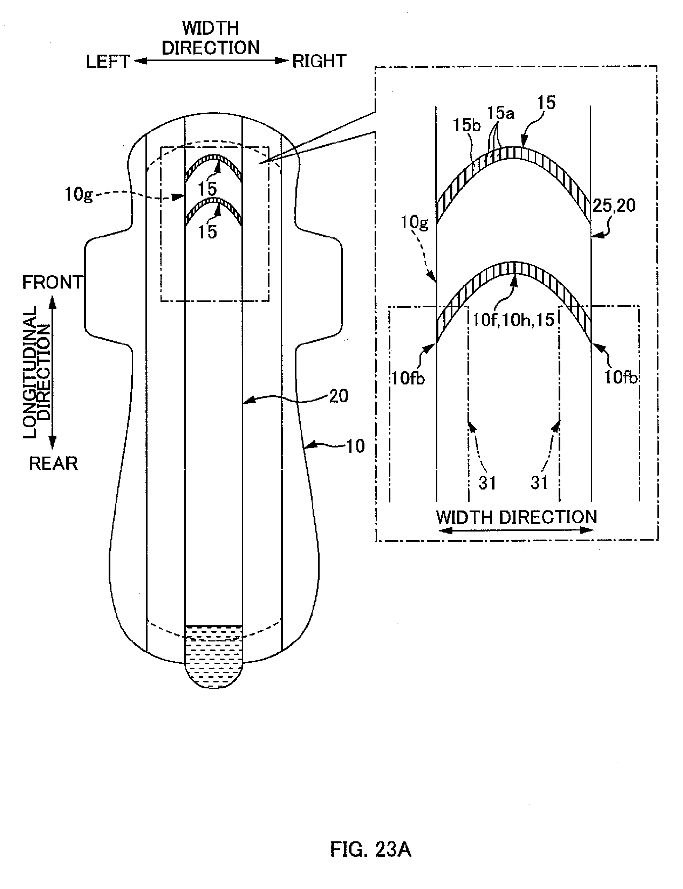

Here, permanent joining refers to an undetachable state in which the top absorbent body 20 and the base absorbent body 10 have been firmly joined, to the extent that in the case where the top absorbent body 20 and the base absorbent body 10 are intentionally separated, damage will inevitably occur to at least any one of the top absorbent body 20 and the base absorbent body 10. As a method of the permanently joining, for example, there are adhesion with hot-melt adhesive with a thick diameter that can perform secure joining, or embossing by heat and pressure bonding in which a deep-grooved embossment section 15 (an embossment in which a groove 15a of a predetermined depth partly contains a portion 15b of an even deeper depth (see an enlarged view in Fig. 23A)) is formed. In here, the former adhesion is adopted which is described later. It should be noted that in the following description, a portion in the front end section 10a of the base absorbent body 10, to which the front-side sealed section 25 of the top absorbent body 20 is permanently joined is referred to as a "permanent joining portion 10g".

Before use, the rear-side sealed section 26 may be temporarily joined to the base absorbent body 10. Here, temporary joining refers to a state in which the base absorbent body 10 and the top absorbent body 20 are weakly joined to the extent that the user can detach and easily separate the top absorbent body 20 from the base absorbent body 10 without impairing the function of the base absorbent body 10 and the top absorbent body 20. As the method of this temporary joining, for example, embossing by pressure bonding is known.

In this temporarily joined state, the rear-side sealed section 26, for example, is protruding about 20 mm to the rear from the outer contour of the base absorbent body 10, so that the user can pick up the rear-side sealed section 26 (see Fig. 1). More specifically, a rear end portion of the rear-side sealed section 26 functions as a pick-up section 26a for being picked up by the user at the time of wearing the absorbent article 1 or the like. Furthermore, as shown in Figs. 9 and 10D, a portion 26b on the front side of the back face (a portion that faces the base absorbent body 10) of the rear-side sealed section 26 is provided with a hook member 27 for engaging the rear end of the top absorbent body 20 with the undergarment 90 at the time of using the absorbent article 1. The hook member 27 is, for example, a male member of a mechanical fastener or the like.

Note that the hook member 27 may be provided in a portion other than the above sealed section 26. For example, the hook member 27 may be provided in a portion that is on the back face side of the top absorbent body 20 and a position at which the pulverized pulp 22 is present on the surface side. At that time, a portion of the base absorbent body 10 facing the hook member 27 may be provided with an engagement member that is to be engaged with the hook member 27. In the case where the hook member 27 is a male member of a mechanical fastener, a female member of the mechanical fastener is used as the engagement member, for example.

Moreover, since the top absorbent body 20 in a worn state is sandwiched in the groove of the buttocks and the like, the top absorbent body 20 can be fixed to the human body even without the hook member 27. In other words, the hook member 27 is not an essential configuration, and may be omitted.

Furthermore, it is desirable that, as shown in Figs. 9 and 10C, in a region on the rear side of the top absorbent body 20, a center section in the width direction thereof is provided with a thin-walled section 22f in which the amount of the pulverized pulp 22 is smaller than in any other portion in the width direction. With such thin-walled section 22f, the top absorbent body 20 is easily folded in a mountain fold, and thus easily placed in the groove of the buttocks and the like of the human body. However, as described above, in the case where the hook member 27 is provided in the portion that is on the back face side of the top absorbent body 20 in which the pulverized pulp 22 is present on the surface side, it is preferable not to form the thin-walled section 22f in the portion in which the hook member 27 is provided. The reason for this is because, in the case where the portion of the top absorbent body 20 in which the hook member 27 is provided is easily folded in a mountain fold, it is difficult for the hook member 27 to engage with the base absorbent body 10. Therefore, for example, in the top absorbent body 20, it is preferable not to form the thin-walled section 22f in and rearward of the portion in which the hook member 27 is provided.

### <Wrapping of Absorbent Article 1>

Fig. 11 is a diagram illustrating how the absorbent article 1 is wrapped. As shown in Fig. 11, in the case where the absorbent article 1 is wrapped, the wing sections 32 on both sides of the absorbent article 1 are each folded toward the surface side, and the protection sheet 35 is provided covering the second displacement-preventing adhesive sections 33 of the wing sections 32 in this folded state from the surface side. Also on the back face 30c of the back face sheet 30, the protection sheet 34 is provided covering the displacement-preventing adhesive section 31. In this state, the absorbent article 1 is folded in three together with a substantially rectangular wrapping sheet 36 that is disposed on the back face side of the absorbent article 1, so that a front end section 1a and a rear end section 1b are on the surface side. In this state where the absorbent article 1 is folded in three, a lead tape 38 provided at the front end of the wrapping sheet 36 is attached to the outer face on the rear end side of the wrapping sheet 36 that has been already folded. After that, in the wrapping sheet 36, edge sections 36c along the longitudinal direction are adhered and sealed to become a package-shape, and thus the absorbent article 1 is in a wrapped state.

### <Wearing of the Absorbent Article 1 on a Human Body>

The absorbent article 1 that has been passed into the hands of the user in this above-described wrapped state is taken out of the wrapping sheet 36 by the user peeling the lead tape 38 and opening the wrapping sheet 36 as shown in Fig. 11.

Then, first, the protection sheet 34 of the absorbent article 1 is peeled, and the displacement-preventing adhesive section 31 on the back face 30c of the back face sheet 30 is exposed (Fig. 2). Then, as shown in Fig. 6, the absorbent article 1 is disposed on the inner face of the undergarment 90 and attached and fixed to the undergarment 90 with the displacement-preventing adhesive section 31. At the same time, the protection sheet 35 on the wing sections 32 is peeled and the second displacement-preventing adhesive sections 33 are exposed, and the wing sections 32 are folded back toward the undergarment 90 side and are attached and fixed to the outer face of the undergarment 90 with the second displacement-preventing adhesive sections 33.

After the absorbent article 1 has been attached and fixed to the undergarment 90 in this manner, the undergarment 90 is pulled up to the human body side, that is, in a state of being worn by the user. Then, as shown in Fig. 7, the pick-up section 26a of the top absorbent body 20 is picked up by the user and the top absorbent body 20 is pulled up. Accordingly, the temporary joining between the base absorbent body 10 and the top absorbent body 20 is released, and the rear end section 20b of the top absorbent body 20 is separated from the base absorbent body 10.

Afterward, due to the user pulling up the pick-up section 26a rearward in the longitudinal direction (substantially the vertical direction), the position of the top absorbent body 20 is adjusted so that the top absorbent body 20 is placed in the groove of the buttocks and the like of the human body and is in close contact. And in the state where the position of the top absorbent body 20 is adjusted, the hook member 27 of the top absorbent body 20 is engaged and fixed to a face on the human body side of the back part of the undergarment 90 or an edge section of the undergarment 90, as shown in Fig. 8. Thereby, the top absorbent body 20 is positioned in a state suitably abutting against the human body.

### <Problems at the time of Wearing the Absorbent Article 1 and Description of the Outline of Present Embodiment for Solving the Problems>

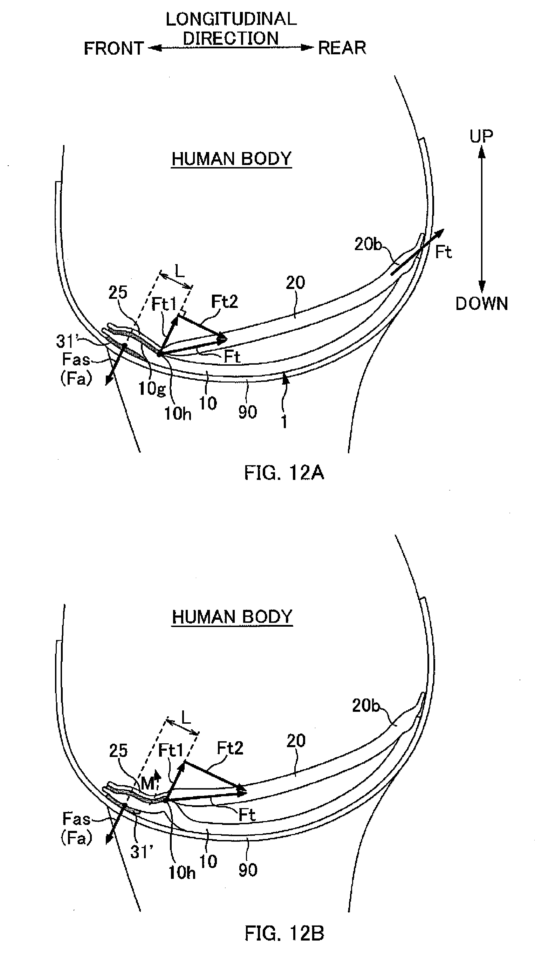

Figs. 12A and 12B are schematic diagrams for illustrating problems at the time of wearing the absorbent article 1, and both are vertical cross-sectional views of the center of the human body in a state where an absorbent article 1 of a comparative example is being worn on the human body.

As described above, at the time of wearing the absorbent article 1 on the human body, the base absorbent body 10 is attached and fixed to the undergarment 90, and in a state where the user wears this undergarment 90, the rear end section 20b (corresponding to an end section) of the top absorbent body 20 is pulled up toward the rear (see Fig. 12A). At this time, a pulling force Ft is transmitted to the base absorbent body 10 via the permanent joining portion 10g (the portion 10g permanently joined to the front-side sealed section 25 of the top absorbent body 20) that is set in the front end section 10a of the base absorbent body 10, and finally acts in a direction in which the base absorbent body 10 is pulled apart from the undergarment 90. Herein, the displacement-preventing adhesive section 31' as described above is formed on the back face 30c of the back face sheet 30 of the base absorbent body 10. Therefore, usually, adhesion force Fa of the displacement-preventing adhesive section 31' counteracts the pulling force Ft as reaction force, and thus the absorbent article 1 is maintained in a state attached and fixed to the undergarment 90 without being detached from the undergarment 90.

However, the pulling force Ft intensively acts especially on a rear end 10h of the permanent joining portion 10g (corresponding to the predetermined portion), that is, the end 10h of the permanent joining portion 10g that is located on the side of the free end of the top absorbent body 20. For this reason, as shown in the comparative example of Fig. 12A, in the case where the displacement-preventing adhesive section 31' is formed only in a predetermined area forward of the rear end 10h of the permanent joining portion 10g, due to the pulling force Ft that acts on the rear end 10h of the permanent joining portion 10g, the displacement-preventing adhesive section 31' is easily detached from the undergarment 90 with relatively little resistance, starting from a position corresponding to the rear end 10h and proceeding toward the front side therefrom, as shown in Fig. 12B. Accordingly, the absorbent article 1 of the comparative example lacks in terms of the stability of adhesion to the undergarment 90.

The reason why the displacement-preventing adhesive section 31' of the comparative example is detached easily as described above can be dynamically-explained as follows, for example. Namely, as shown in Fig. 12A, the pulling force Ft that acts on the rear end 10h of the permanent joining portion 10g of the base absorbent body 10 can be divided into a component force Ft1 that acts in the normal direction of adhesive face of the displacement-preventing adhesive section 31' and a component force Ft2 that acts in a direction parallel to the adhesive face. Of these component forces Ft1 and Ft2 acting in the two directions, it is the former normal-direction component force Ft1 that contributes to the detachment of the base absorbent body 10 from the undergarment 90. More specifically, the normal-direction component force Ft1 acts on the base absorbent body 10 as a detaching force for detaching the base absorbent body 10 from the undergarment 90, and the adhesion force Fa of the displacement-preventing adhesive section 31' counteracts this detaching force Ft1.

However, as shown in the comparative example of Fig. 12A, in the case where the displacement-preventing adhesive section 31' is formed only in a predetermined area that is forward of the rear end 10h of the permanent joining portion 10g, the position of action of a resultant Fas of the adhesion forces Fa, which acts as the reaction force of the detaching force Ft1, is inevitably located forward than the rear end 10h of the permanent joining portion 10g. Then, as shown in Fig. 12A, the resultant Fas of the adhesion forces Fa and the detaching force Ft1 produce a couple while being separated by a distance L between the aforementioned position of action and the rear end 10h. As a result, based on above couple, a counterclockwise moment M indicated by a dashed arrow in Fig. 12B acts on a portion in the vicinity of the rear end 10h of the permanent joining portion 10g in the base absorbent body 10. Due to this moment M, the displacement-preventing adhesive section 31' is easily detached from the undergarment 90, starting at the position corresponding to the rear end 10h.

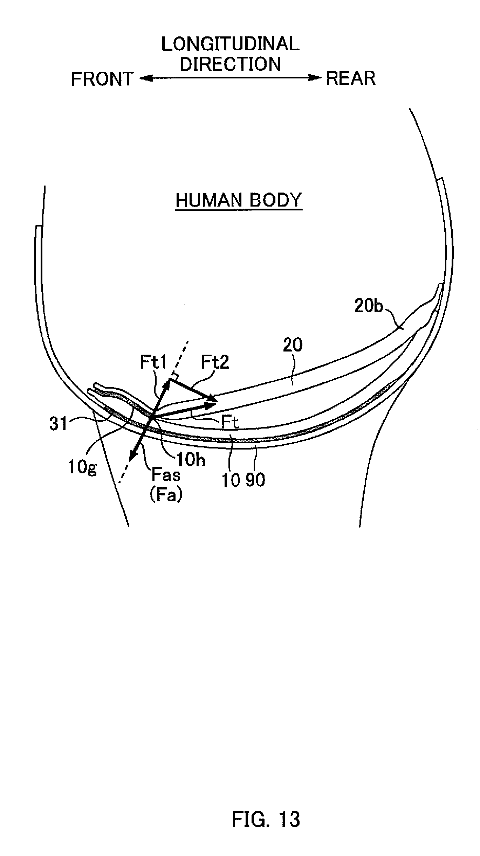

In contrast, in the case of the present embodiment shown in Fig. 13, the displacement-preventing adhesive section 31 (corresponding to adhesive section) is included continuously spanning the rear end 10h (corresponding to an end on the free end side) of the permanent joining portion 10g from front to rear (i.e., both sides in the longitudinal direction). More specifically, the displacement-preventing adhesive section 31 is formed not only in the predetermined area forward of the rear end 10h, in which this predetermined area is extended rearward, but also in a predetermined area rearward of the rear end 10h. Thus, the resultant Fas of the adhesion forces Fa of the displacement-preventing adhesive sections 31 can be made to act on a position that corresponds to the rear end 10h of the permanent joining portion 10g, as shown in Fig. 13. In other words, it becomes possible to make the resultant Fas of the adhesion forces Fa act in the opposite direction of the detaching force Ft1 while aligned in the same straight line. Then, the couple is hardly generated in the portion in the vicinity of the rear end 10h of the permanent joining portion 10g in the base absorbent body 10, and the moment M that might detach the displacement-preventing adhesive section 31 starting from the position corresponding to the rear end 10h is unlikely to be generated. As a result, the displacement-preventing adhesive section 31 becomes unlikely to be detached from the undergarment 90.

### <Formation Area of the Displacement-Preventing Adhesive Section 31 according to First Embodiment>

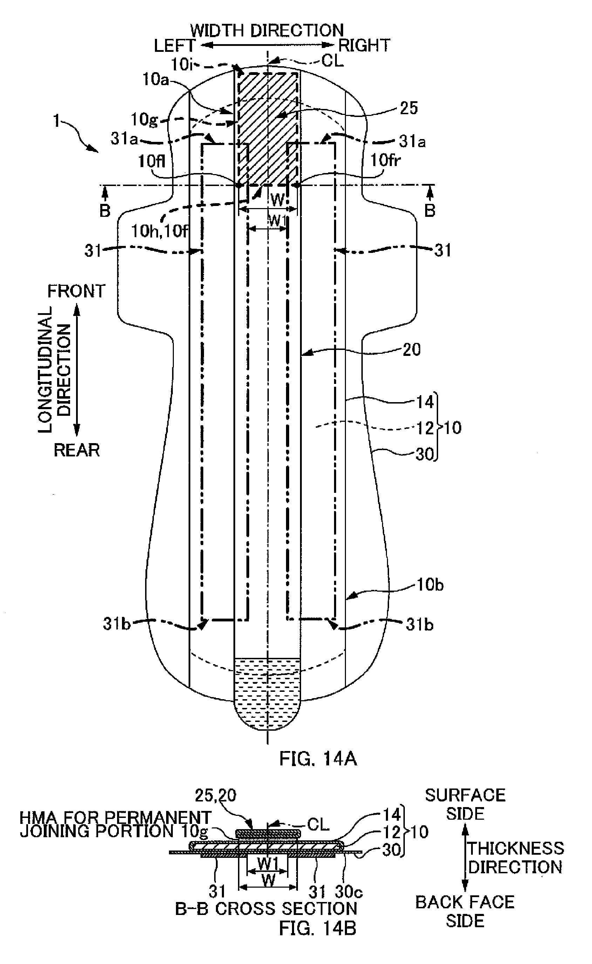

First, before a description of the formation area of displacement-preventing adhesive section 31 according to first embodiment, the portion 10g (i.e., the permanent joining portion 10g) of the base absorbent body 10 to which the front-side sealed section 25 (corresponding to a part of the absorbent body) of the top absorbent body 20 is joined is described. Fig. 14A is a plan view of an absorbent article 1 of the first embodiment, and Fig. 14B is a cross-sectional view taken along line B-B of Fig. 14A.

As shown in Figs. 14A and 4, the permanent joining portion 10g is set in the center of the front end section 10a in the width direction of the base absorbent body 10, and the shape of the permanent joining section 10g viewed from above is substantially rectangular almost the same shape as the front-side sealed section 25 of the top absorbent body 20. A hot-melt adhesive HMA is applied to a region (a portion indicated by the oblique lines in Fig. 14A) within the external outline of the permanent joining portion 10g. And by overlapping and adhering the front-side sealed section 25 of the top absorbent body 20 with the permanent joining portion 10g, the top absorbent body 20 is permanently joined to the front end section 10a of the base absorbent body 10.

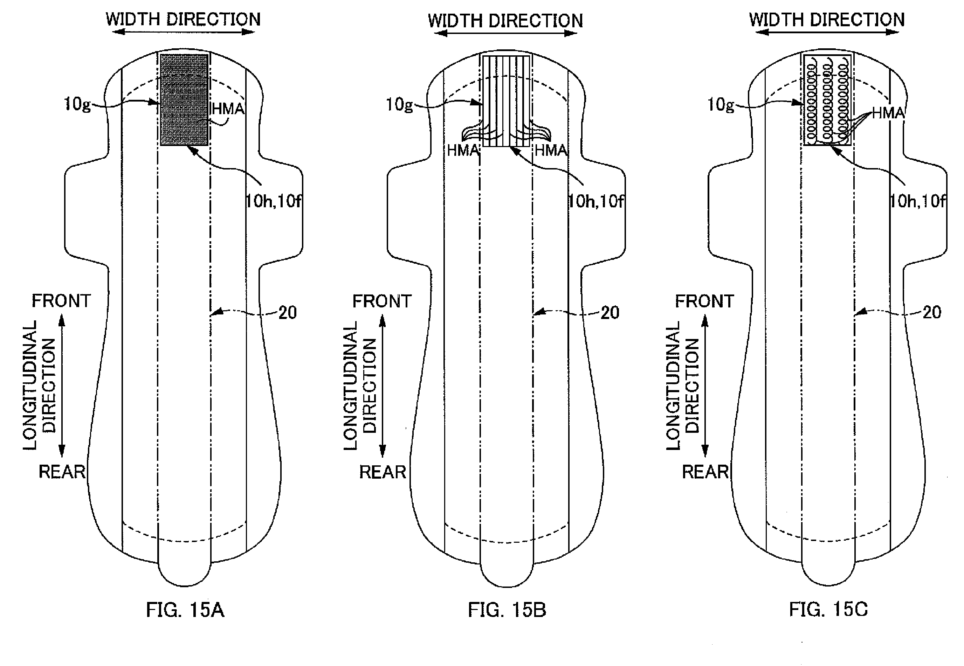

Figs. 15A to 15C show examples of the application patterns of the hot-melt adhesive HMA. In Figs. 15A to 15C, the top absorbent body 20 is virtually indicated with a chain double-dashed line. Examples of the application patterns of the hot-melt adhesive HMA include a pattern in which the adhesive is solidly applied to all over the permanent joining portion 10g as shown by shading in Fig. 15A, a pattern in which the adhesive is applied in a plurality of straight lines along the longitudinal direction as shown in Fig. 15B, and furthermore, a pattern in which the adhesive is applied in a plurality of continuous spiral curves extending in the longitudinal direction as shown in Fig. 15C. However, in any application pattern, it is preferable that the positions of the rear end 10h of the hot-melt adhesive HMA in the longitudinal direction are aligned through the entire length of the rear end 10h in the width direction. More specifically, it is preferable that a rear end edge 10f of the permanent joining portion 10g is parallel to the width direction as shown in the examples of Figs. 15A to 15C. The reason for this is because in the case where the positions of the rear end 10h of the permanent joining portion 10g in the longitudinal direction are displaced forward or rearward depending on the positions in the width direction thereof, the above-described pulling force Ft intensively acts on the rear end 10h, of all portions in the rear end edge 10f, that is located rearward relative to the other portions, and this becomes a cause of local breakage at the time of pulling up the top absorbent body 20.

Next, the formation area of the displacement-preventing adhesive sections 31 of the first embodiment is described. It should be noted that in the following description, the width direction is also referred to as a right-and-left direction.

As shown in Figs. 14A and 14B, the displacement-preventing adhesive section 31 is formed by solidly applying a hot-melt adhesive to the back face 30c of the back face sheet 30 in the base absorbent body 10. In an example shown in Figs. 14A and 14B, the displacement-preventing adhesive sections 31 that are pair on the right and left sides in the width direction are disposed axisymmetrically relative to the center line CL in the width direction of the base absorbent body 10, while being separated from each other by a predetermined distance W1 in the width direction. It should be noted that the center line CL in the width direction of the base absorbent body 10 is same as the center line CL in the width direction of the top absorbent body 20. Therefore, the pair of displacement-preventing adhesive sections 31 also can be said as being disposed axisymmetrically relative to the center line CL in the width direction of the top absorbent body 20.

The right and left displacement-preventing adhesive sections 31 each have the same substantially rectangular shape elongated in the front-and-rear direction when viewed from above. A front end edge 31a of each of the displacement-preventing adhesive sections 31 is located between (at about the midpoint, in the example shown in Figs. 14A and 14B) a front end edge 10i and the rear end edge 10f of the permanent joining section 10g, and a rear end edge 31b of each of the displacement-preventing adhesive sections 31 is located in a rear end section 10b of the base absorbent body 10. Accordingly, both of the right and left displacement-preventing adhesive sections 31 are included continuously spanning the rear end edge 10f of the permanent joining portion 10g from front to rear. Thus, the above-described moment M shown in Fig. 12B is unlikely to be produced. As a result, the right and left displacement-preventing adhesive sections 31 are unlikely to be detached from the undergarment 90 in a state where the absorbent article 1 is worn.

Moreover, as described above, the right and left displacement-preventing adhesive sections 31 are disposed axisymmetrically relative to the center line CL in the width direction of the top absorbent body 20. Thus, the right and left displacement-preventing adhesive sections 31 can counteract the above-described pulling force Ft that acts on the rear end edge 10f of the permanent joining portion 10g in a well-balanced manner with respect to the width direction.

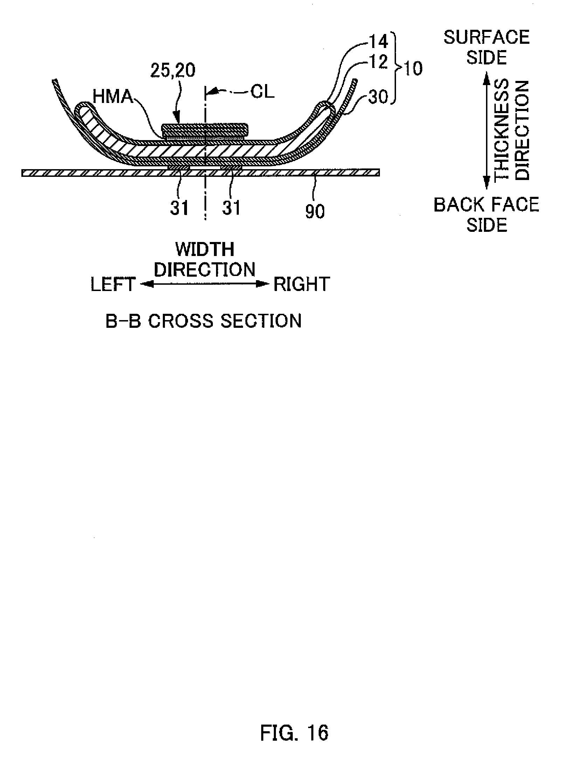

Furthermore, the left displacement-preventing adhesive section 31 extends leftward beyond a left end 10fl (corresponding to an end in the width direction of the end edge) of the rear end edge 10f of the permanent joining portion 10g, while the right displacement-preventing adhesive section 31 extends rightward beyond a right end 10fr (corresponding to an end in the width direction of the end edge) of the rear end edge 10f of the permanent joining portion 10g. That is to say, the right and left displacement-preventing adhesive sections 31 are also provided outside the rear end edge 10f (corresponding to an end edge) of the permanent joining portion 10g in the width direction. Thus, with these displacement-preventing adhesive sections 31, in the case where the pulling force Ft acts on the permanent joining portion 10g, as shown in Fig. 16, both end sections of the base absorbent body 10 in the width direction can be effectively prevented from being separated and folded back from the undergarment 90.

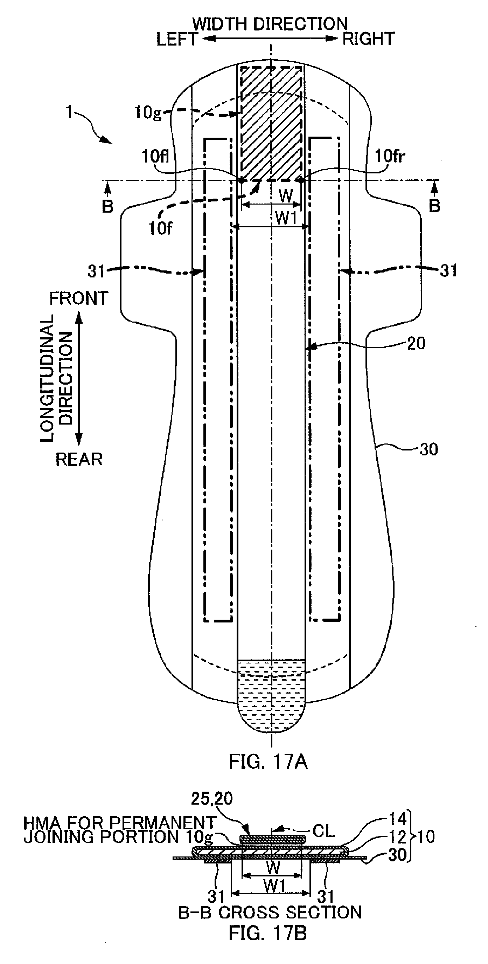

Figs. 17A and 17B are explanatory diagrams of a modification of the first embodiment. Fig. 17A shows a plan view of an absorbent article 1 of the modification, and Fig. 17B shows a cross-sectional view taken along line B-B of Fig. 17A.

The main difference between the above-described first embodiment and the modification thereof is the difference in the size of distance W1 between the right and left displacement-preventing adhesive sections 31. More specifically, in the case of the first embodiment, as shown in Figs. 14A and 14B, the distance W1 between the right and left displacement-preventing adhesive sections 31 is narrower than the width W of the rear end edge 10f of the permanent joining portion 10g, however in the case of this modification, as shown in Figs. 17A and 17B, the distance W1 between the right and left displacement-preventing adhesive sections 31 is wider than the width W of the rear end edge 10f of the permanent joining portion 10g.

In here, preferably, it is advantageous to adopt the first embodiment. This is because it is possible to make the moment that acts to detach the displacement-preventing adhesive section 31 in the width direction smaller in the first embodiment.

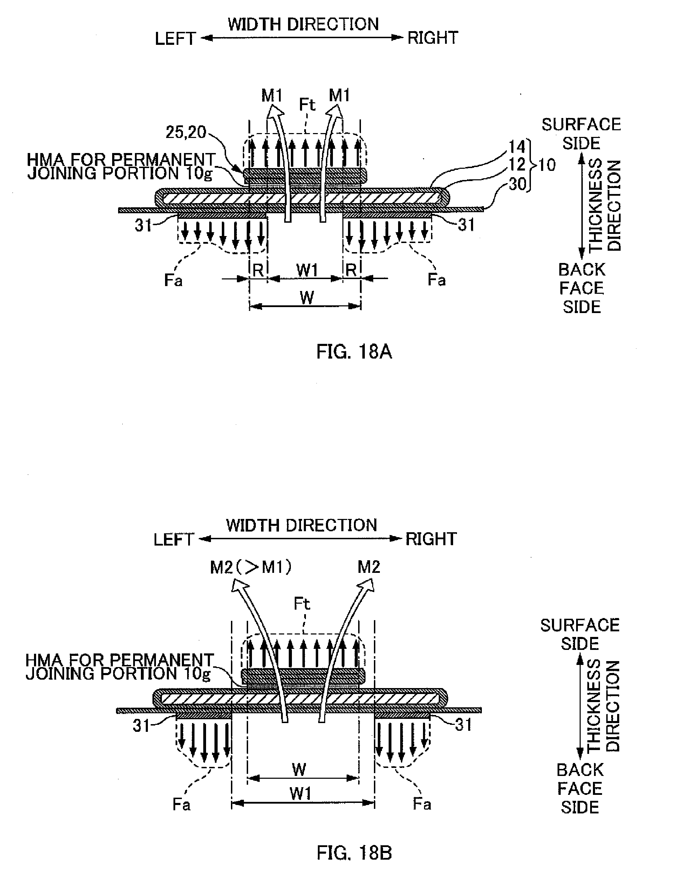

Figs. 18A and 18B are vertical cross-sectional views of the absorbent article 1 for explaining the reason for this in detail. Fig. 18A shows the case of the above-described first embodiment, and Fig. 18B shows the case of the above-described modification. It should be noted that in both of Figs. 18A and 18B, the pulling force Ft and the adhesion force Fa counteracting the pulling force Ft are represented as distributed loads.

In both cases of the first embodiment shown in Fig. 18A and the modification shown in Fig. 18B, the displacement-preventing adhesive sections 31 are not provided in a center portion in the width direction of the base absorbent body 10. Thus, moments M1 and M2 as respectively shown in Figs. 18A and 18B act on the center portion based on a couple of the pulling force Ft and the adhesion force Fa. And the moments M1 and M2 act to detach the displacement-preventing adhesive sections 31 along the width direction.

Herein, in the case of the modification shown in Fig. 18B, the distance W1 between the right and left displacement-preventing adhesive sections 31 is wider than the width W of the rear end edge 10f of the permanent joining portion 10g. Therefore the distributed loads of the pulling force Ft and the distributed loads of the adhesion force Fa produce couples with respect to every load. Thus, large moments M2 are formed based on those couples.

In contrast, in the case of the first embodiment shown in Fig. 18A, the distance W1 between the right and left displacement-preventing adhesive sections 31 is narrower than the width W of the rear end edge 10f of the permanent joining portion 10g. That is to say, the displacement-preventing adhesive sections 31 and the permanent joining portion 10g have overlapping sections R with respect to the width direction. No couple is generated based on the distributed loads of the pulling force Ft and the distributed loads of the adhesion force Fa that act on the overlapping sections R. Thus, the moment M1 that acts on the center portion of the base absorbent body 10 become smaller than the moment M2 that may be produced in the above-described modification. As a result, the displacement-preventing adhesive sections 31 of the first embodiment become more unlikely to be detached than those of the modification.

In other words, as in the first embodiment, the absorbent article 1 has superior adhesion stability in the case where the distance W1 between the right and left displacement-preventing adhesive sections 31 is narrower than the width W of the rear end edge 10f of the permanent joining portion 10g. In still other words, from the viewpoint of adhesion stability, it is more preferable that the displacement-preventing adhesive sections 31 are included continuously spanning, in the width direction, the ends 10fl and 10fr of the rear end edge 10f of the permanent joining portion 10g in the width direction as shown in Figs. 14A and 14B.

### Second Embodiment

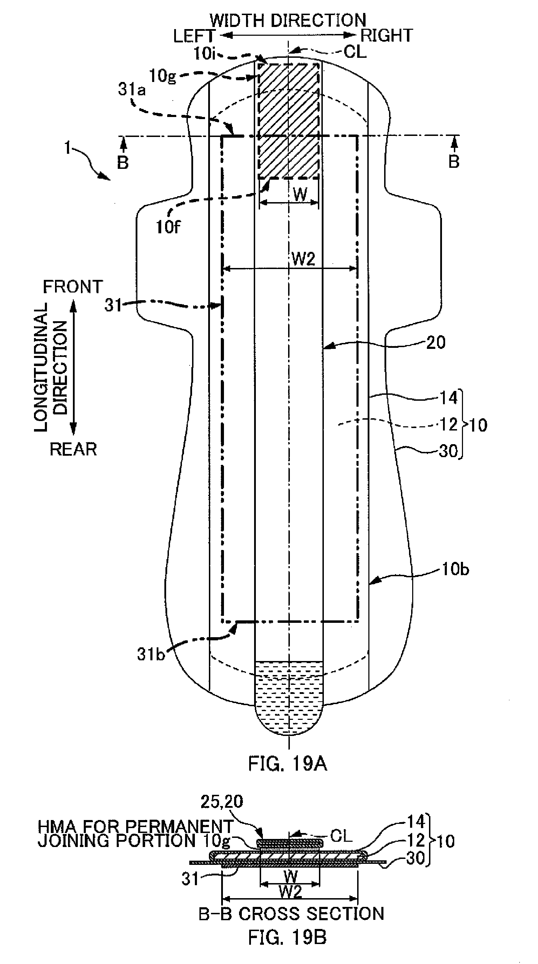

Figs. 19A and 19B are explanatory diagrams of a formation area of displacement-preventing adhesive sections 31 according to second embodiment. Fig. 19A shows a plan view of an absorbent article 1 of the second embodiment, and Fig. 19B shows a cross-sectional view taken along line B-B of Fig. 19A.

In the first embodiment, as shown in Figs. 14A and 14B, the pair of displacement-preventing adhesive sections 31 at left and right are disposed while being separated from each other by the distance W1 in the width direction. However, in the second embodiment, a hot-melt adhesive for preventing displacement is solidly applied to the part of distance W1 between the right and left displacement-preventing adhesive sections 31. As a result, the right and left displacement-preventing adhesive sections 31 are united to form a single continuous region 31 as shown in Figs. 19A and 19B, and the first embodiment and the second embodiment differ with respect to this point.

More specifically, as shown in Fig. 19A, the displacement-preventing adhesive section 31 of the second embodiment is a single region having a substantially rectangular shape when viewed from above. A front end edge 31a of the displacement-preventing adhesive section 31 is located between (at about the midpoint, in the example shown in Fig. 19A) a front end edge 10i and a rear end edge 10f of a permanent joining portion 10g, and a rear end edge 31b of the displacement-preventing adhesive section 31 is located in a rear end section 10b of a base absorbent body 10. Moreover, in the width direction, the displacement-preventing adhesive section 31 is wider than the rear end edge 10f of the permanent joining portion 10g, or in other words, extends beyond the right and left ends of the rear end edge 10f in the width direction.

Thus, with such displacement-preventing adhesive section 31 of the second embodiment, the following effects can be obtained in addition to the above-described effects of the first embodiment and the modification thereof. In the second embodiment, the displacement-preventing adhesive section 31 is formed even in the center portion of the base absorbent body 10 in the width direction, therefore the moment (e.g., the moment M1 in Fig. 18A) produced in the first embodiment and the modification thereof that detach the displacement-preventing adhesive section 31 along the width direction can be generally extinguished.

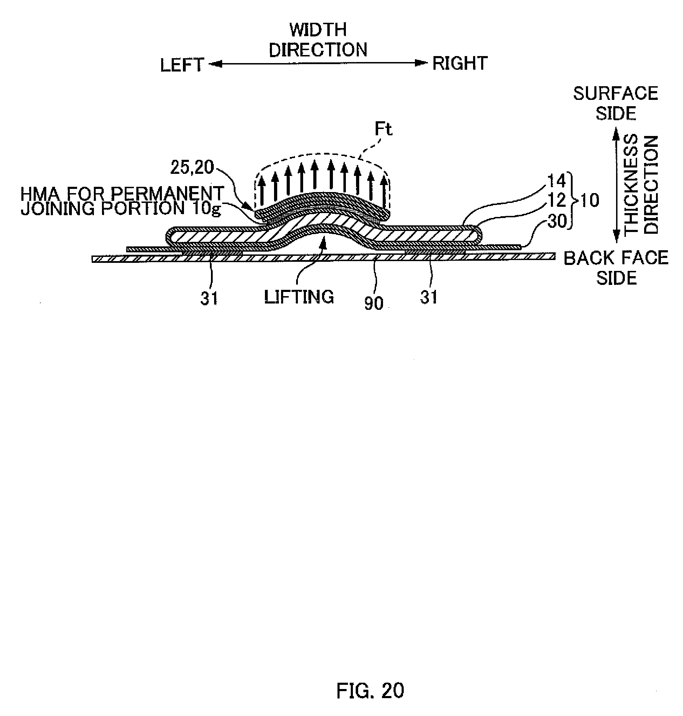

Moreover, the displacement-preventing adhesive section 31 in the center portion in the width direction directly and coaxially counteracts pulling force Ft that acts on the rear end edge 10f of the permanent joining portion 10g, therefore the center portion of the base absorbent body 10 in the width direction can be effectively prevented from being detached and lifted from the undergarment 90 as shown in Fig. 20.

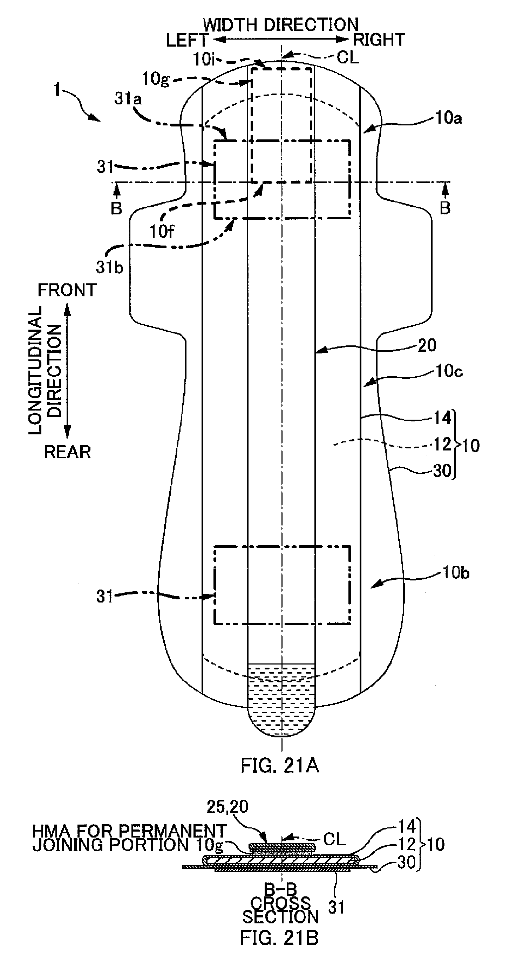

Figs. 21A and 21B are explanatory diagrams of a modification of the second embodiment. Fig. 21A shows a plan view of an absorbent article 1 of the modification, and Fig. 21B shows a cross-sectional view taken along line B-B of Fig. 21A.

The main difference between the above-described second embodiment and the modification thereof is, in the modification of the second embodiment a displacement-preventing adhesive section 31 is not formed in a center section 10c of a base absorbent body 10 in the longitudinal direction, whereby the displacement-preventing adhesive section 31 is divided into two sections, that is, a front displacement-preventing adhesive section 31 and a rear displacement-preventing adhesive section 31. It should be noted that the size of the displacement-preventing adhesive section 31 in the width direction is the same as that in the above-described second embodiment.

As shown in Fig. 21A, the front displacement-preventing adhesive section 31 has a substantially rectangular shape when viewed from above. A front end edge 31a of the front displacement-preventing adhesive section 31 is located between (at about the midpoint, in the example shown in Fig. 21A) a front end edge 10i and a rear end edge 10f of a permanent joining portion 10g, while a rear end edge 31b is located rearward of the rear end edge 10f of the permanent joining portion 10g. Accordingly, the front displacement-preventing adhesive section 31 is included continuously spanning the rear end edge 10f of the permanent joining portion 10g from front to rear, and thus becomes unlikely to be detached from the undergarment 90 at the time of wearing the absorbent article 1.

Also, the rear displacement-preventing adhesive section 31 also has a substantially rectangular shape when viewed from above, and is provided in a rear end section 10b of the base absorbent body 10. Thus, in cooperation with the above-described front displacement-preventing adhesive section 31 provided in the front end section 10a of the base absorbent body 10, the rear displacement-preventing adhesive section 31 can stably adhere and fix the absorbent article 1 to the undergarment 90 over the entire length of the absorbent article 1 in the longitudinal direction.

### Third Embodiment

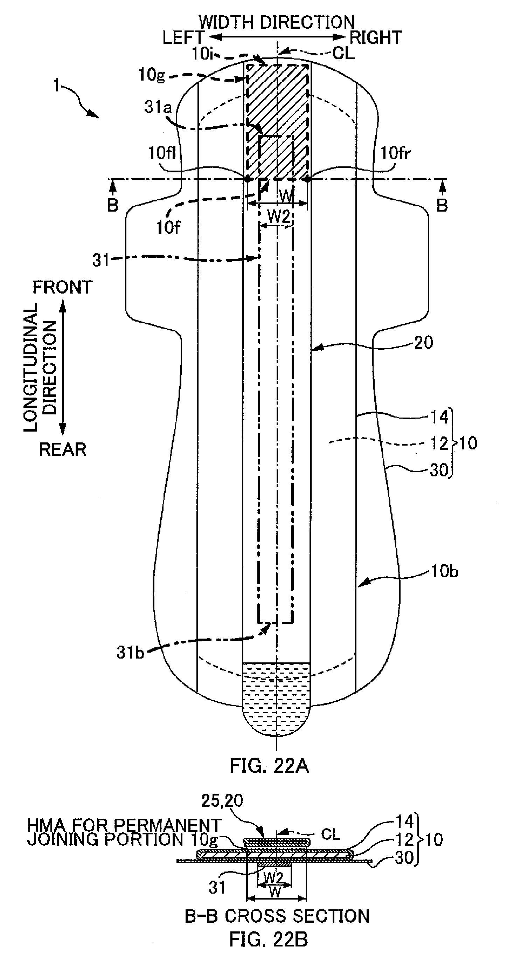

Figs. 22A and 22B are explanatory diagrams of a formation area of a displacement-preventing adhesive section 31 according to third embodiment. Fig. 22A shows a plan view of an absorbent article 1 of the third embodiment, and Fig. 22B shows a cross-sectional view taken along line B-B of Fig. 22A.

In the above-described second embodiment, as shown in Figs. 19A and 19B, the width W2 of the displacement-preventing adhesive section 31 is wider than the width W of the rear end edge 10f of the permanent joining portion 10g. However, in the third embodiment, width W2 of a displacement-preventing adhesive section 31 is narrower than width W of a rear end edge 10f of a permanent joining portion 10g, and the second embodiment and the third embodiment differ with respect to this point.

More specifically, as shown in Figs. 22A and 22B, the displacement-preventing adhesive section 31 of the third embodiment does not extend outward in the right-and-left direction beyond a left end 10fl and a right end 10fr of the rear end edge 10f of the permanent joining portion 10g. Due to this configuration, the amount of hot-melt adhesive to be used can be reduced.

It should be noted that the formation area of the displacement-preventing adhesive section 31 with respect to the longitudinal direction is the same as that in the second embodiment, therefore a description thereof will be omitted.

### Other Embodiments

In the description above, embodiments of the invention were described. However, the invention is not limited to these embodiments, and modifications as described below are possible.

In the above embodiments, the hot-melt adhesive was solidly applied (i.e., applied to all over the intended area without leaving any gap) in order to form the displacement-preventing adhesive section 31. However, the application pattern is not limited to this. More specifically, any application pattern other than the above-described solid application pattern may be adopted, as long as the hot-melt adhesive is applied continuously spanning the rear end edge 10f of the permanent joining portion 10g from front to rear. For example, the hot-melt adhesive may be applied in the form of a plurality of straight lines along the longitudinal direction, or may be applied in the form of a plurality of helical curved lines continuing in the longitudinal direction.

In the above embodiments, the hot-melt adhesive was given as an example of the displacement-preventing adhesive section 31. However, this hot-melt adhesive is not intended to be limiting. And other types of adhesives may be used as long as the base absorbent body 10 can be adhered and fixed to the undergarment 90. Furthermore, the base absorbent body 10 may be engaged and fixed to the undergarment 90 using something other than the adhesive, for example, a male member of a mechanical fastener.

In the above embodiments, as shown in Figs. 15A to 15C, the rear end edge 10f of the permanent joining portion 10g is parallel to the width direction. That is to say, the position of the rear end 10h of the permanent joining portion 10g in the longitudinal direction is aligned across the width direction, but this is not a limitation, and a configuration without this alignment may be adopted. For example, as shown in Fig. 23A, in the case where the front-side sealed section 25 of the top absorbent body 20 is permanently joined to the front end section 10a of the base absorbent body 10 by a pair of deep-grooved embossment sections 15 at the front and rear having a boomerang shape, the rear end edge 10f of the permanent joining portion 10g becomes the rear deep-grooved embossment section 15. In this case, both end portions of the rear deep-grooved embossment section 15 in the width direction are located rearward of a center portion thereof. That is to say, the position of the rear end 10h of the permanent joining portion 10g is not aligned across the width direction.

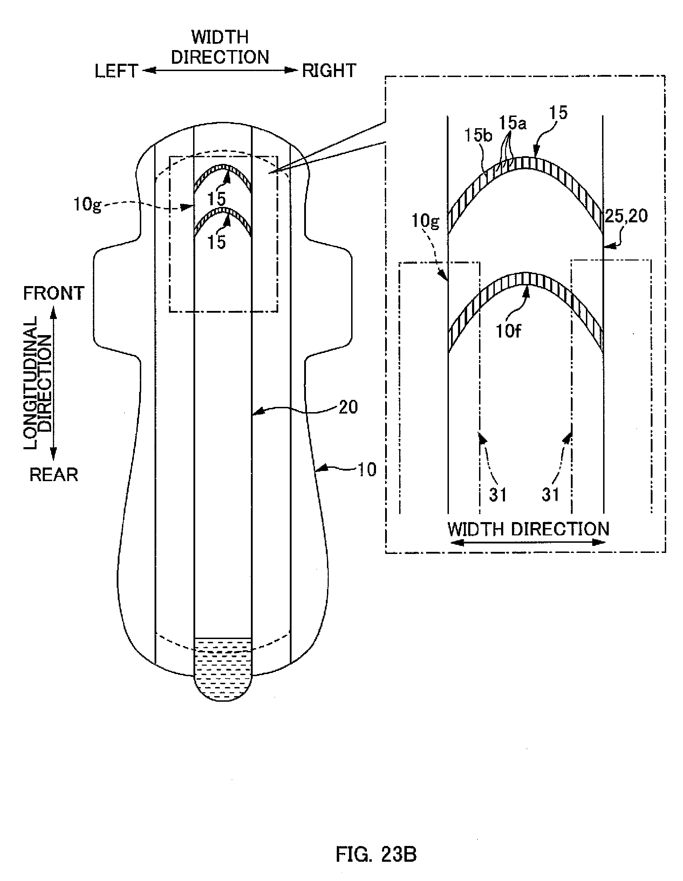

Herein, in such a case, as long as the displacement-preventing adhesive sections 31 are included continuously spanning at least a rearmost end 10fb of the rear end edge 10f of the permanent joining portion 10g (i.e., an end 10fb that is closest to the free end of the top absorbent body 20 of the rear end edge 10f) from front to rear, as shown in an enlarged view in Fig. 23A, the above-described effect of making the displacement-preventing adhesive sections 31 unlikely to be detached can be obtained. However, to enhance this effect even more, it is desirable that the displacement-preventing adhesive section 31 is included continuously spanning the rear end edge 10f of the permanent joining portion 10g from front to rear as shown in Fig. 23B.

In the above embodiments, as the absorbent article 1, an example in which only the pick-up section 26a of the top absorbent body 20 is projected from the external outline of the base absorbent body 10 in an unfolded state is described (see Fig. 1). However, there is no limitation to this.

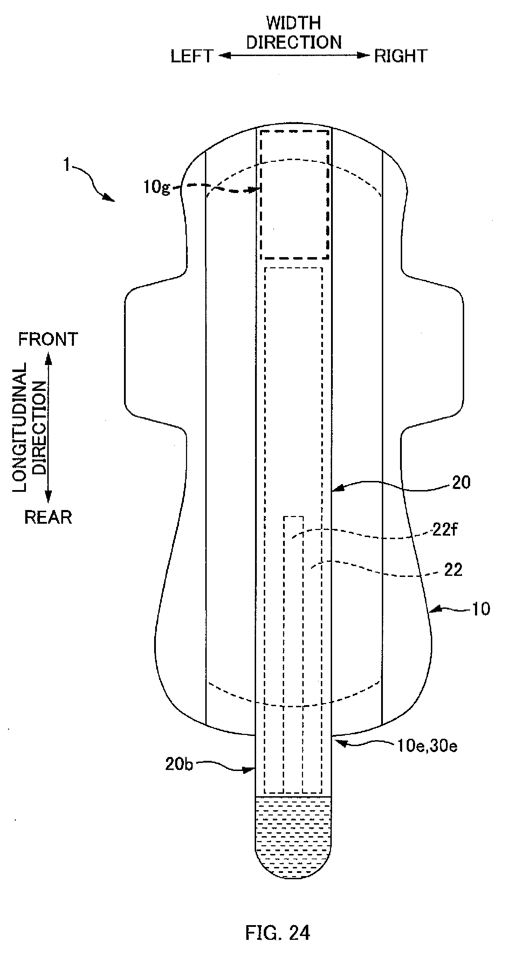

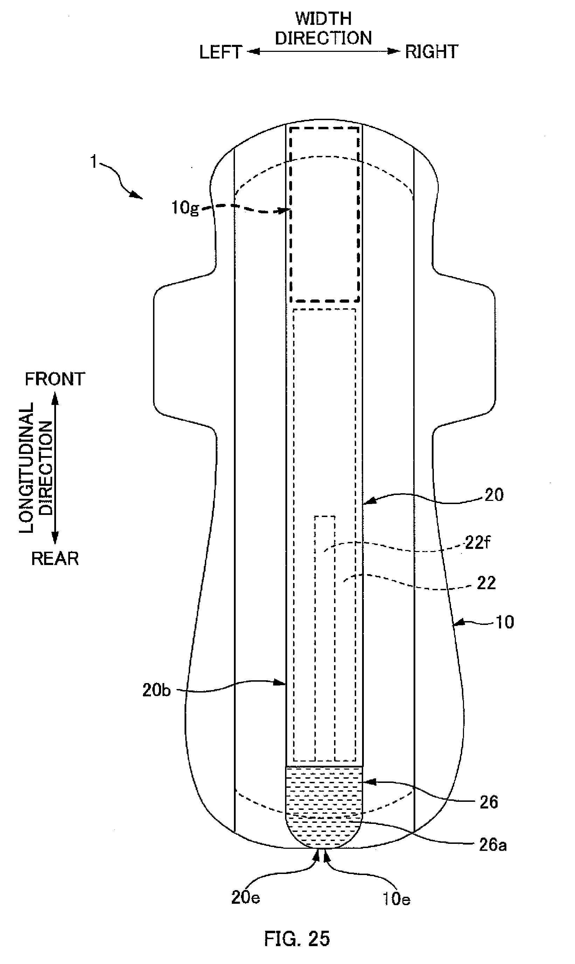

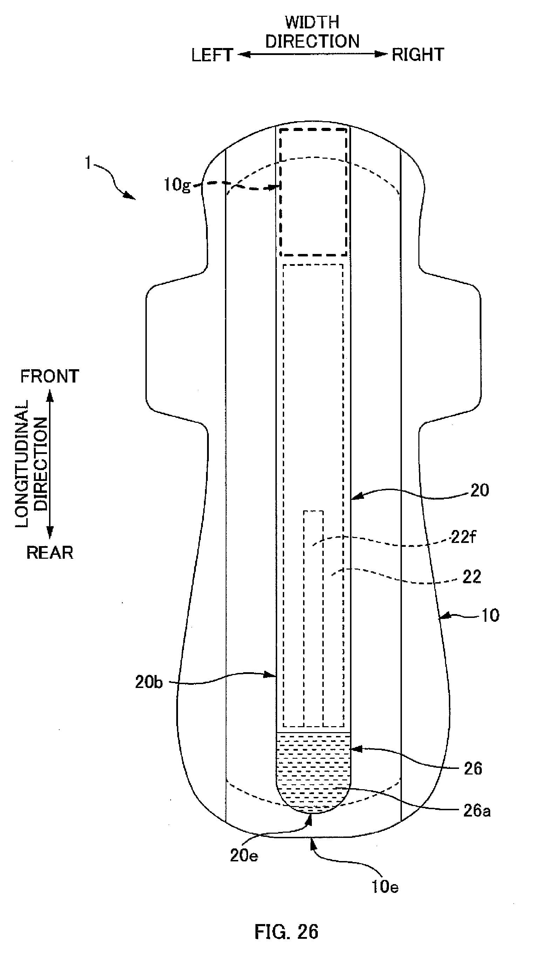

For example, as shown in a plan view of Fig. 24, in an unfolded state, not only the pick-up section 26a of the top absorbent body 20 but also a portion of the top absorbent body 20 to which the pulverized pulp 22 extends may be projected from the external outline of the base absorbent body 10. Also, as shown in Fig. 25, the position of the extreme edge of the pick-up section 26a as the rear end edge 20e of the top absorbent body 20 may be aligned with the position of the rear end edge 10e of the external outline of the base absorbent body 10. Furthermore, as shown in Fig. 26, the rear end edge 20e of the top absorbent body 20 may be located forward of the rear end edge 10e of the external outline of the base absorbent body 10.

In the foregoing embodiments, as shown in Fig. 5, the front-side sealed section 25 of the top absorbent body 20 is permanently joined to the permanent joining portion 10g set in the front end section 10a of the base absorbent body 10, and thus the rear end section 20b of the top absorbent body 20 can rotate in the front-and-rear direction as a free end with the front-side sealed section 25 as a fulcrum. However, the position of the permanent joining portion 10g and the end section as the free end are not limited to this configuration.

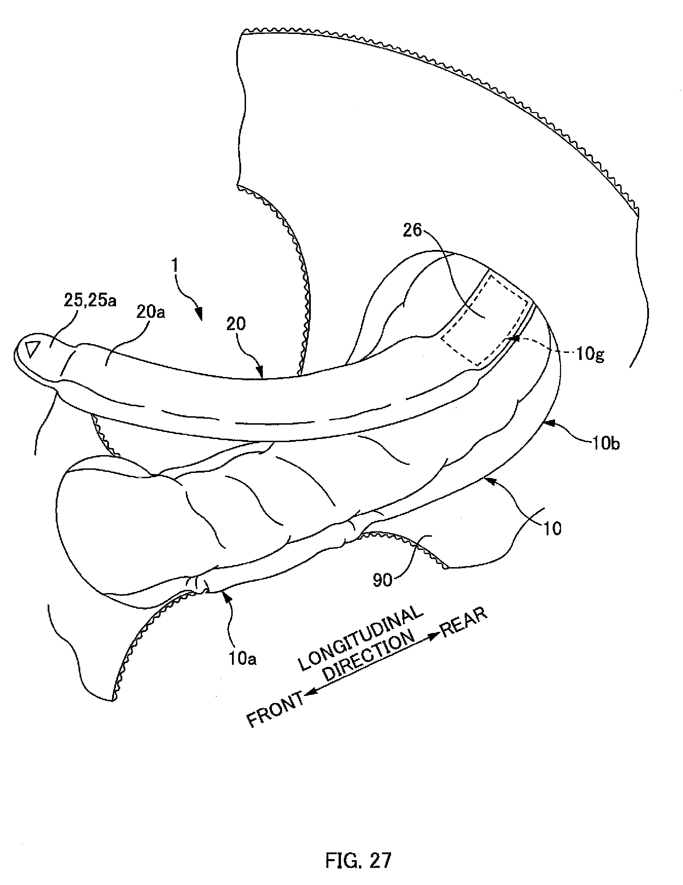

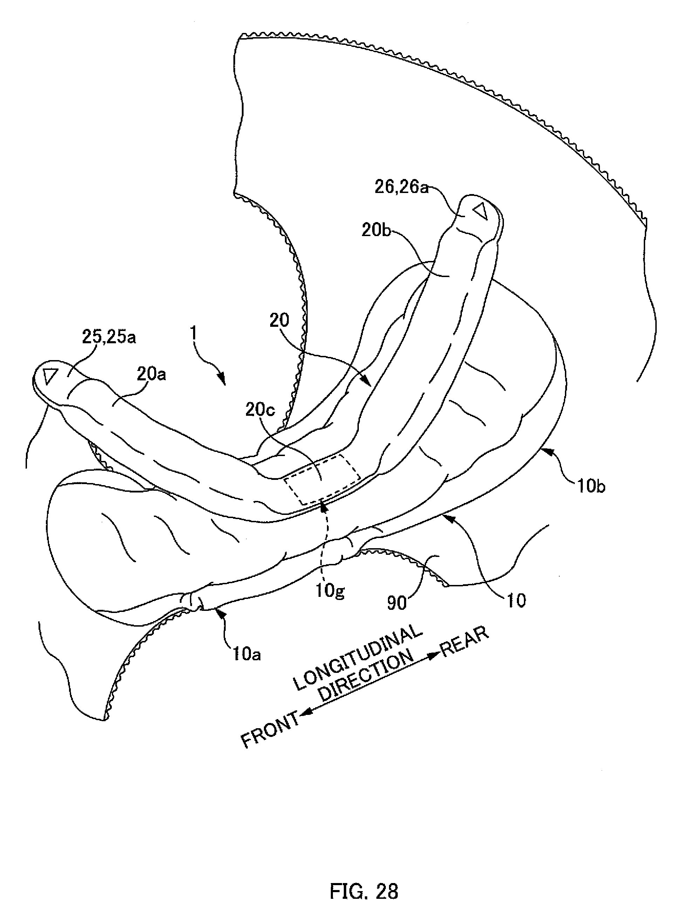

For example, as shown in Fig. 27, it is also possible that the rear-side sealed section 26 of the top absorbent body 20 is permanently joined to the permanent joining portion 10g set in the rear end section 10b of the base absorbent body 10, and thus the front end section 20a of the top absorbent body 20 can rotate in the front-and-rear direction as a free end with the rear-side sealed section 26 as a fulcrum. Furthermore, as shown in Fig. 28, it is also possible that a center section 20c of the top absorbent body 20 in the longitudinal direction is permanently joined to the permanent joining portion 10g set in a center portion of the base absorbent body 10 in the longitudinal direction, and thereby both of the front end section 20a and the rear end section 20b of the top absorbent body 20 can serve as free ends that can rotate in the front-and-rear direction with the center section 20c as a fulcrum. It goes without saying that in the former case, a pick-up section 25a that is to be used when the top absorbent body 20 is pulled up may be formed in the front-side sealed section 25, and in the latter case, pick-up sections 25a and 26a that are to be used when the top absorbent body 20 is pulled up may be formed in both of the front-side sealed section 25 and the rear-side sealed section 26.

In the foregoing embodiments, as shown in Fig. 4, only the front-side sealed section 25 of the top absorbent body 20 was intended to be permanently joined. However, there is no limitation to this. For example, the intended portion to be permanently joined may not only be the front-side sealed section 25, but rather may be extended beyond the rear end of the front-side sealed section 25 to a portion of the top absorbent body 20 in which the pulverized pulp 22 is present. More specifically, the length of the permanent joining portion 10g in the longitudinal direction may be longer than the front-side sealed section 25 of the top absorbent body 20.

In the foregoing embodiments, as shown in Fig. 3, a configuration in which the base absorbent body 10 as "the main body of the absorbent article" includes the absorbent body base material 12 containing the pulverized pulp 12a and the like was described as an example. However, there is no limitation to this. For example, "the main body of the absorbent article" may be the above-described configuration of the base absorbent body 10 from which the absorbent body base material 12 is removed. However, in this configuration, the base absorbent body 10 does not include the pulverized pulp 12a and the like. Therefore this configuration is inferior as a whole in terms of the fluid absorption ability.

In the foregoing embodiments, the base absorbent body 10 includes the single absorbent body base material 12 in the center in the width direction thereof. However, there is no limitation to this. For example, side absorbent bodies may be respectively provided, along the longitudinal direction, at both end sections of the base absorbent body 10 in the width direction. Alternatively, instead of these side absorbent bodies, solid gathers for preventing movement of fluid in the width direction of the base absorbent body 10 may be respectively provided in the both end sections.

In the foregoing embodiments, a sanitary napkin is disclosed as the absorbent article 1. However, there is no limitation to this, and the absorbent article 1 may be any sort of article as long as it can be adhered to the undergarment and absorb fluid discharged from the human body.

## Claims

1. An absorbent article that is worn on a human body and used, comprising:
an absorbent body having a fluid-absorbing member that absorbs fluid and having a longitudinal direction, a width direction, and a thickness direction;
a main body whose face on the human body side is overlapped with the absorbent body in the thickness direction; and
an adhesive section for adhering the main body to an undergarment, the adhesive section being formed on a face on the opposite side of the face on the human body side of the main body,
wherein a part of the absorbent body in the longitudinal direction is joined to a predetermined portion of the main body, and an end section of the absorbent body in the longitudinal direction can be moved separably from the main body as a free end with respect to the part, and
wherein the adhesive section is included continuously spanning, in the longitudinal direction, an end of the predetermined portion, the end being on the free end side in the longitudinal direction.

2. An absorbent article according to claim 1, wherein
the adhesive section is included continuously spanning, in the longitudinal direction, at least an end that is positioned closest to the free end side in the end.

3. An absorbent article according to claim 1 or 2, wherein
the end of the predetermined portion on the free end side defines an end edge having a predetermined width in the width direction orthogonal to the longitudinal direction, and
the adhesive section is included continuously spanning the end edge in the longitudinal direction.

4. An absorbent article according to claim 3, wherein
the adhesive section is disposed at least outside the end edge in the width direction.

5. An absorbent article according to claim 4, wherein
an absorbent body and a main body are overlapped while matching their center in the width direction, and
the adhesive section is axisymmetrically disposed relative to a center line of the absorbent body in the width direction.

6. An absorbent article according to claim 4 or 5, wherein
the adhesive section is included continuously spanning, in the width direction, an end of the end edge in the width direction.

7. An absorbent article according to claim 5, wherein
the adhesive section is included continuously spanning an end edge in the width direction.
